# EUROPÄISCHE PATENTSCHRIFT

(11) **EP 3 558 085 B1**
(45) Veröffentlichungstag und Bekanntmachung des Hinweises auf die Patenterteilung: **13.04.2022**
(21) Anmeldenummer: 17816644.3
(22) Anmeldetag: 07.12.2017
(51) Int. Cl.: A47L 15/44

(54) **BEDUFTUNG UND/ODER DESODORIERUNG EINES REINIGUNGSGERÄTES**
PERFUMING AND/OR DEODORISING A CLEANING DEVICE
DIFFUSION DE PARFUM ET/OU DÉSODORISATION D'UN APPAREIL DE NETTOYAGE

(30) Priorität: 21.12.2016 DE 102016225838
(43) Veröffentlichungstag der Anmeldung: 30.10.2019
(73) Patentinhaber: Henkel AG & Co. KGaA, 40589 Düsseldorf (DE)
(72) Erfinder: KESSLER, Arnd, 40789 Monheim am Rhein (DE); WEBER, Thomas, 41541 Dormagen (DE); ZIPFEL, Johannes, 40593 Düsseldorf (DE); NITSCH, Christian, 40591 Düsseldorf (DE); ZÜCHNER, Lars, 40764 Langenfeld (DE); FRANKE, Nadine, 50668 Köln (DE); WAWER, Georg, 1040 Wien (AT); MÜLLER, Alexander, 40789 Monheim (DE); WICK, Wolfgang, 41542 Dormagen (DE)
(86) Internationale Anmeldenummer: PCT/EP2017/081859
(87) Internationale Veröffentlichungsnummer: WO 2018/114364

(56) Entgegenhaltungen:
- EP-A1- 1 843 693
- DE-A1-102007 015 246
- ES-A1- 2 574 566
- KR-A- 20040 000 101
- KR-A- 20100 022 695
- US-A1- 2010 139 366

## Beschreibung

### Gebiet

Ausführungsformen der Erfindung betreffen die Beduftung und/oder die Desodorierung eines Reinigungsgerätes, insbesondere die Beduftung und/oder die Desodorierung des Innenraums eines Reinigungsgerätes.

### Hintergrund

Ein bekanntes Problem bei Reinigungsgeräten, wie etwa Geschirrspülmaschinen, ist, dass in Abhängigkeit der Schmutzfracht (z.B. verunreinigtes Geschirr) und der Dauer dessen Aufbewahrung sich unangenehme Gerüche entwickeln können. Erst wenn ein Reinigungsprogramm der Geschirrspülmaschine gestartet wird, wird der unangenehme Geruch durch die entsprechende Reinigung beseitig. Oftmals wird beispielsweise aus Energieverbrauchsgründen ein Reinigungsprogramm einer Geschirrspülmaschine erst gestartet, wenn die Kapazität für zu reinigendes Geschirr der Geschirrspülmaschine nahezu vollständig genutzt wird.

Zur Verminderung der Geruchsbelastung bzw. zu deren Überdeckung sind Beduftungsmittel für Geschirrspülmaschinen bekannt. Diese Beduftungsmittel umfassen in der Regel Trägermaterialien und auf diesen Trägermaterialien befindliche Duftstoffe, die kontinuierlich an die Umgebung abgegeben werden.

Nachteilig an dieser bekannten Lösung ist, dass die Menge des abgegeben Beduftungsmittels konstant und kontinuierlich ist, bis das Beduftungsmittel vollständig verbraucht ist. Die Beduftung erfolgt vollkommen unabhängig von der tatsächlichen geruchlichen Belastung. Es ist entsprechend keine Anpassung an die tatsächliche geruchliche Belastung möglich. Ferner können gewisse unangenehme Gerüche durch eine derartige Beduftung nicht effektiv beseitigt werden, da mit einer konstanten Abgabe von Beduftungsmittel lediglich ein Überdecken des unangenehmen Geruchs möglich ist.

Dokument DE102007015246A1 beschreibt ein Verfahren zur Abgabe eines Duftstoffes im Inneren einer Waschmaschine in Form von Nebel. Die Abgabe wird durch die Detektion von Molekülen, die mit schlechtem Geruch assoziiert sind, mittels eines Geruchssensors ausgelöst.

### Allgemeine Beschreibung einiger beispielhafter Ausführungsformen

Vor dem Hintergrund des dargestellten Standes der Technik ist es somit Aufgabe, die beschriebenen Probleme zumindest teilweise zu verringern oder zu vermeiden, das heißt insbesondere eine Möglichkeit bereitzustellen, dass in Abhängigkeit einer vorhandenen Geruchsbelastung eine angepasste Beseitigung der Geruchsbelastung möglich ist.

Diese Aufgabe wird gegenständlich durch ein beispielhaftes Verfahren gelöst, das folgendes umfasst:
- Erfassen von ersten Informationen oder Erhalten von erfassten ersten Informationen indikativ für eine Geruchsbelastung im Innenraum eines Reinigungsgerätes, wobei die ersten Informationen von mindestens einem Geruchssensor erfasst werden, wobei die erfassten ersten Informationen indikativ für zumindest die Dynamik der Geruchsbelastung im Innenraum des Reinigungsgerätes sind;
- Erzeugen einer Beduftungs- und/oder Desodorierungsinformation zumindest teilweise basierend auf den erfassten oder erhaltenen ersten Informationen oder Ausgeben der erfassten ersten Informationen zum Erzeugen einer derartigen Beduftungs- und/oder Desodorierungsinformation;
- Auslösen einer Beduftung und/oder Desodorierung mittels mindestens eines Aktuators für Beduftungs- und/oder Desodorierungsmittel basierend auf der erzeugten Beduftungs- und/oder Desodorierungsinformation.

In einem Ausführungsbeispiel umfasst das Verfahren das Erhalten der erfassten ersten Informationen und das Erzeugen der Beduftungs- und/oder Desodorierungsinformation. In einem anderen Ausführungsbeispiel umfasst das Verfahren das Erfassen der ersten Informationen und das Ausgeben der erfassten ersten Informationen zum Erzeugen der Beduftungs- und/oder Desodorierungsinformation.

Weiter wird ein Dosiergerät zur Positionierung im Innenraum eines Reinigungsgerätes offenbart, das Dosiergerät umfassend:
- mindestens eine Kartusche zur Bevorratung von mindestens einer Zubereitung eines Beduftungs- und/oder Desodorierungsmittels;
- eine Sensoreinheit umfassend mindestens einen Geruchssensor, wobei der Geruchssensor eingerichtet ist erste Informationen zu erfassen oder erfasste erste Informationen indikativ für eine Geruchsbelastung im Innenraum des Reinigungsgerätes zu erhalten, wobei die erfassten ersten Informationen indikativ für zumindest die Dynamik der Geruchsbelastung im Innenraum des Reinigungsgerätes sind;
- eine Steuereinheit, wobei mittels der Steuereinheit eine Beduftungs- und/oder Desodorierungsinformation basierend auf von der Sensoreinheit erfassten ersten Informationen erzeugbar ist;
- einen Aktuator zum Auslösen einer Beduftung und/oder Desodorierung basierend auf der erzeugten Beduftungs- und/oder Desodorierungsinformation, wobei mindestens eine Zubereitung eines Beduftungs- und/oder Desodorierungsmittels freisetzbar ist,
- optional eine Kommunikationsschnittstelle.

Weiter wird eine Vorrichtung offenbart, die zur Ausführung und/oder Steuerung des Verfahrens eingerichtet ist oder jeweilige Mittel zur Ausführung und/oder Steuerung der Schritte des Verfahrens umfasst. Dabei können entweder alle Schritte des Verfahrens gesteuert werden, oder alle Schritte des Verfahrens ausgeführt werden, oder ein oder mehrere Schritte gesteuert und ein oder mehrere Schritte ausgeführt werden. Ein oder mehrere der Mittel können auch durch die gleiche Einheit ausgeführt und/oder gesteuert werden. Beispielsweise können ein oder mehrere der Mittel durch einen oder mehrere Prozessoren gebildet sein.

Weiter wird eine Vorrichtung offenbart, die zumindest einen Prozessor und zumindest einen Speicher, der Programmcode beinhaltet, umfasst, wobei der Speicher und der Programmcode eingerichtet sind, mit dem zumindest einen Prozessor eine Vorrichtung (beispielsweise die Vorrichtung mit dem Prozessor und dem Speicher) dazu zu veranlassen, zumindest das Verfahren auszuführen und/oder zu steuern. Dabei können entweder alle Schritte des Verfahrens gesteuert werden, oder alle Schritte des Verfahrens ausgeführt werden, oder ein oder mehrere Schritte gesteuert und ein oder mehrere Schritte ausgeführt werden.

Weiter wird ein System offenbart, das eine oder mehrere Vorrichtungen umfasst, die eingerichtet sind zur Ausführung und/oder Steuerung des Verfahrens oder Mittel zur Ausführung und/oder Steuerung der Schritte des Verfahrens aufweisen. Dabei weist vorzugsweise mindestens eine Vorrichtung (1, 2, 3, 50, 51) eine Kommunikationsschnittstelle auf. Dabei können entweder alle Schritte des Verfahrens gesteuert werden, oder alle Schritte des Verfahrens ausgeführt werden, oder ein oder mehrere Schritte gesteuert und ein oder mehrere Schritte ausgeführt werden.

Weiter wird ein Computerprogramm offenbart, das Programmanweisungen umfasst, die einen Prozessor zur Ausführung und/oder Steuerung des Verfahrens veranlassen, wenn das Computerprogramm auf dem Prozessor läuft. Unter einem Prozessor sollen in dieser Spezifikation unter anderem Kontrolleinheiten, Mikroprozessoren, Mikrokontrolleinheiten wie Mikrocontroller, digitale Signalprozessoren (DSP), Anwendungsspezifische Integrierte Schaltungen (ASICs) oder Field Programmable Gate Arrays (FPGAs) verstanden werden. Dabei können entweder alle Schritte des Verfahrens gesteuert werden, oder alle Schritte des Verfahrens ausgeführt werden, oder ein oder mehrere Schritte gesteuert und ein oder mehrere Schritte ausgeführt werden. Das Computerprogramm kann beispielsweise über ein Netzwerk wie das Internet, ein Telefon- oder Mobilfunknetz und/oder ein lokales Netzwerk verteilbar sein. Das Computerprogramm kann zumindest teilweise Software und/oder Firmware eines Prozessors sein. Es kann gleichermaßen zumindest teilweise als Hardware implementiert sein. Das Computerprogramm kann beispielsweise auf einem computerlesbaren Speichermedium gespeichert sein, z.B. einem magnetischen, elektrischen, elektromagnetischen, optischen und/oder andersartigen Speichermedium. Das Speichermedium kann beispielsweise Teil des Prozessors sein, beispielsweise ein (nicht-flüchtiger oder flüchtiger) Programmspeicher des Prozessors oder ein Teil davon. Das Speichermedium ist beispielsweise gegenständlich, also greifbar, und/oder nicht-transitorisch.

Diese sechs Aspekte weisen u.a. die nachfolgend beschriebenen - teilweise beispielhaften - Eigenschaften auf.

Unter einer Beduftung ist eine merkliche Freisetzung von Riechstoffen zu verstehen. Die Riechstoffe können von menschlichen olfaktorischen Rezeptoren wahrgenommen und vom Gehirn eines Menschen als Duft übersetzt werden. Riechbare Stoffe sind Aktivstoffe, die in Tieren oder Pflanzen gebildet oder synthetisch hergestellt werden. Damit ein Stoff als Geruch wahrnehmbar ist, muss der Stoff flüchtig sein. Die Flüchtigkeit ist eine Funktion von Molekülgröße und der Wechselwirkung mit anderen Molekülen. Bei einer Molmasse von etwa 300 g/mol ist die Grenze der Flüchtigkeit in etwa erreicht (dies entspricht etwa 20 Kohlenstoffatomen). Für den Menschen angenehme Riechstoffe finden sich im Bereich von 8 bis 16 Kohlenstoffatomen.

Unter einer Desodorierung ist eine geeignete Maßnahme zu verstehen, um einen störenden Schlechtgeruch zu beseitigen. Dies kann beispielsweise durch physikalische Mittel (z.B. durch Adsorption/Absorption) geschehen oder durch chemische, nicht reaktive Mittel. Diese chemischen, nicht reaktiven Mittel sind in der Lage, den störenden Schlechtgeruch für einen Menschen auszublenden, zu maskieren oder durch Eigengeruch zu überdecken. Es ist jedoch nicht zwingend für eine Desodorierung erforderlich, eine (aktive) Geruchswirkung herzustellen.

Eine Geschirrspülmaschine repräsentiert beispielsweise ein Reinigungsgerät.

Es erfolgt ein Erfassen von ersten Informationen. Alternativ erfolgt ein Erhalten von erfassten ersten Informationen. Die ersten Informationen sind indikativ für eine Geruchsbelastung im Innenraum eines Reinigungsgerätes. Die ersten Informationen geben also beispielsweise eine quantitative und/oder qualitative Größe über eine Geruchsbelastung im Innenraum eines Reinigungsgerätes an.

Die ersten Informationen werden mit mindestens einem Geruchssensor erfasst. Der mindestens eine Geruchssensor ist beispielsweise ein Stickstoff-empfindlicher Sensor. Mittels des mindestens einen Geruchssensors ist beispielsweise die Anwesenheit eines Amins erfassbar. Der mindestens eine Geruchssensor ist beispielsweise ein elektrochemischer Sensor. Mittels des mindestens einen Geruchssensors ist beispielsweise die Anwesenheit von Ethanol erfassbar. Der mindestens eine Geruchssensor erfasst die Anwesenheit von Ethanol in der Umgebung des mindestens einen Geruchssensors und erzeugt entsprechend der Konzentration des Ethanols ein elektrisches Signal.

Die Anwesenheit von Ethanolm, die beispielsweise von dem mindestens einen Geruchssensor erfassbar ist, ist indikativ für eine mikrobielle Verunreinigung. Beispielsweise erzeugen Hefen Ethanol als Stoffwechselprodukt. Mindestens ein Geruchssensor, der als elektrochemischer Sensor ausgebildet ist - im Folgenden auch als Ethanolsensor bezeichnet - weist beispielsweise in Abhängigkeit von einer Ethanol-Konzentration in der Gasphase eine unterschiedliche Ansprechempfindlichkeit auf. Dabei ändert sich der Term dA/dT (welcher von dem erzeugten elektrischen Signal repräsentiert wird; "Strom zu Zeit-Kurve"; Ableitung Strom [Ampere] über die Zeit [T]) hin zu kleineren Werten mit abnehmender Ethanol-Konzentration. Entsprechend verändert sich die Strom zu Zeit-Kurve hin zu flacheren Steigungen pro Zeiteinheit. Daraus lassen sich beispielsweise Rückschlüsse auf die vorhandene absolute Konzentration ziehen, aber auch eine zeitliche Konzentrationsänderung beobachten. Eine derartige zeitliche Konzentrationsänderung tritt insbesondere bei einer mikrobiellen Verunreinigung verursacht durch eine mikrobielle Besiedlung auf. Insofern ist das Erfassen der Ethanol-Konzentration mittels des mindestens einen Geruchssensors im Innenraum des Reinigungsgerätes (insbesondere zwischen zwei Reinigungszyklen des Reinigungsgerätes) insbesondere indikativ für eine mikrobielle Belastung bzw. für die Geruchsbelastung im Innenraum des Reinigungsgerätes.

Überraschenderweise hat sich herausgestellt, dass mittels mindestens eines Geruchssensors, der als Ethanolsensor ausgebildet ist, neben der Anwesenheit von Ethanol auch weitere Alkohole, Ester, Ketone und Aldehyde erfassbar sind. Dabei verändert sich das von dem mindestens einen Geruchssensor erzeugte elektrische Signal entsprechend.

Mittels der von dem mindestens einen Geruchssensor erfassten ersten Informationen ist eine Beduftungs- und/oder Desodorierungsinformation erzeugbar, die eine Bewertung des olfaktorischen Zustands des Innenraums eines Reinigungsgerätes ermöglicht. Neben der Erfassbarkeit der Anwesenheit und Konzentration von Ethanol kann multidimensional die Anwesenheit verschiedenster Riechstoffe und Schlechtgerüche erkannt werden. Dabei ist lediglich die Empfindlichkeit des mindestens einen Geruchssensors (insbesondere ein Ethanolsensor) je nach Substanz beschränkt. Für den Fall, dass der mindestens eine Geruchssensor als Ethanolsensor ausgebildet ist, kann eine Empfindlichkeitsabstufung nach organischen Gruppen beispielsweise wie folgt definiert werden:

R-OH >> RCHO > RCOOR >>> RCO.

Alkohole werden beispielsweise deutlich besser erkannt als Aldehyde. Aldehyde werden wiederrum besser erkannt als Ester. Ester werden deutlich besser erkannt als Ketone.

Wenn das Verfahren gemäß dem ersten Aspekt das Erhalten erfasster erster Informationen umfasst, sind die erfassten ersten Informationen beispielsweise wie zuvor beschrieben erfasst worden.

Es wird zumindest teilweise basierend auf den erfassten oder erhaltenen ersten Informationen eine Beduftungs- und/oder Desodorierungsinformation erzeugt. Die Beduftungs- und/oder Desodorierungsinformation umfasst beispielsweise eine Menge, eine Frequenz, einen Zeitpunkt, oder eine Kombination hiervon. Auf Basis dieser vorstehenden Werte kann beispielsweise das Auslösen einer Beduftung und/oder Desodorierung des Innenraums des Reinigungsgerätes erfolgen.

Insbesondere vor dem Hintergrund, dass in der Regel im Innenraum eines Reinigungsgerätes von verschiedensten Lebensmitteln verursachte Geruchsbelastungen herrschen, liegt ein Substanzgemisch in der Gasphase vor. Die von diesem Substanzgemisch ausgehende Geruchsbelastung wird beispielsweise mittels des einen mindestens einen Geruchssensors erfasst. Aufgrund der von verschiedensten Lebensmitteln verursachten Geruchsbelastungen sieht eine beispielhafte Ausgestaltung vor, dass das Erzeugen der Beduftungs- und/oder Desodorierungsinformationen einen Vergleich zwischen den erfassten ersten Informationen mit einem vordefinierten Schwellwert umfasst.

Beispielsweise ist der vordefinierte Schwellwert entsprechend der geringsten Empfindlichkeitsabstufung des mindestens einen Geruchssensors definiert. Es erfolgt ein Vergleich zwischen den erfassten ersten Informationen und dem vordefinierten Schwellwert. Überschreiten die erfassten ersten Informationen den vordefinierten Schwellwert, wird beispielsweise eine Beduftungs- und/oder Desodorierungsinformation erzeugt werden. Die Beduftungs- und/oder Desodorierungsinformation ist dabei beispielsweise indikativ für eine olfaktorische Änderung im Innenraum des Reinigungsgerätes. Die olfaktorische Änderung kann beispielsweise noch unter einer Wahrnehmungsschwelle für einen Menschen liegen, da die mit der olfaktorischen Änderung verbundene Geruchsbelastung im Innenraum des Reinigungsgerätes unter einer individuellen Empfindlichkeitsschwelle liegt. Entsprechend kann die Beduftungs- und/oder Desodorierungsinformation als Mengenangabe lediglich eine geringe Menge umfassen, so dass bei kleinen Werten der von dem mindestens einen Geruchssensor erfassten ersten Informationen eine geringe Menge Beduftungs- und/oder Desodorierungsmittel freigesetzt wird durch das Auslösen der Beduftung und/oder Desodorierung. Bei größeren und/oder steigenden Änderungswerten, die von dem mindestens einen Geruchssensor erfasst werden, kann als Mengenangabe, die von der Beduftungs- und/oder Desodorierungsinformation umfasst ist, eine (z.B. automatische) Steigerung der freizusetzenden Menge an Beduftungs- und/oder Desodorierungsmittel oder eine größere Menge angegeben werden. Alternativ kann auch eine Frequenzangabe von der Beduftungs- und/oder Desodorierungsinformation umfasst sein, welche im Rahmen des Auslösens der Beduftung und/oder Desodorierung eine geringe Menge an Beduftungs- und/oder Desodorierungsmittel wiederholt (z.B. in zeitlichen Abständen) freisetzt.

Gemäß einer Ausführungsform aller Aspekte sind die erfassten ersten Informationen und/oder der vordefinierte Schwellwert indikativ für zumindest einen der folgenden Parameter:
- Intensität der Geruchsbelastung im Innenraum des Reinigungsgerätes;
- Art der Geruchsbelastung im Innenraum des Reinigungsgerätes;
- Dynamik der Geruchsbelastung im Innenraum des Reinigungsgerätes;
- oder eine Kombination hiervon.

Unter der Intensität der Geruchsbelastung im Innenraum des Reinigungsgerätes wird insbesondere die von einem Nutzer empfundene Geruchsbelastung, die durch die Amplitude des von dem mindestens einen Geruchssensor erzeugten elektrischen Signals repräsentiert wird, verstanden. Je höher die Intensität der Geruchsbelastung ist, umso schneller und deutlicher kann ein Nutzer die Geruchsbelastung erfassen.

Unter der Art der Geruchsbelastung im Innenraum des Reinigungsgerätes wird insbesondere verstanden, wonach der von der Geruchsbelastung hervorgerufene Geruch primär riecht, also welche Geruchsdimension vorliegt. Beispielsweise kann die Art der Geruchsbelastung eine qualitative Aussage umfassen, z.B. die Geruchsbelastung ist von der Art "saure Milch" oder "Essig", um ein paar exemplarische Beispiele zu nennen.

Unter der Dynamik einer Geruchsbelastung im Innenraum des Reinigungsgerätes wird insbesondere verstanden, wie schnell die Intensität einer Geruchsbelastung über eine Zeitspanne zunimmt.

Gemäß einer beispielhaften Ausführungsform aller Aspekte umfasst das Verfahren:
- Erhalten einer Nutzereingabe betreffend eine Anpassung des vordefinierten Schwellwertes;
- Adaptieren des vordefinierten Schwellwertes basierend auf der erhaltenen Nutzereingabe.

Das Erfassen der Nutzereingabe umfasst beispielsweise das maschinelle Erfassten einer Information, beispielsweise eine Eingabe auf einer Eingabevorrichtung, die seitens des Nutzers durchgeführt wird, z.B. mittels einer Tasteneingabe auf einer Tastatur, und/oder auf einer berührungsempfindlichen Anzeigevorrichtung. Das Erfassen der Nutzereingabe kann beispielsweise mit Mitteln zur Erfassung der Nutzereingabe stattfinden. Wird die Nutzereingabe erhalten, ist die erhaltene Nutzereingabe beispielsweise wie zuvor beschrieben erfasst worden.

Gemäß einer beispielhaften Ausführungsform aller Aspekte umfasst das Verfahren:
- Erfassen von zweiten Informationen oder Erhalten von erfassten zweiten Informationen indikativ für einen Lichteintritt und/oder eine Temperatur im Innenraum des Reinigungsgerätes, wobei die zweiten Informationen von mindestens einem Lichtsensor und/oder mindestens einem Temperatursensor erfasst werden.

Der mindestens eine Lichtsensor erfasst beispielsweise den Lichteintritt in den Innenraum des Reinigungsgerätes, der beispielsweise durch ein Beladen des Reinigungsgerätes bei Öffnen einer Tür des Reinigungsgerätes hervorgerufen wird. Nach einem Beladungsvorgang wird in der Regel die Tür des Reinigungsgerätes wieder geschlossen. Der Zeitraum der Öffnung und die Veränderung des Lichteintritts, der durch den mindestens einen Lichtsensor erfassbar ist und beispielsweise durch ein Herausziehen und Einschieben der zur Halterung von zu reinigendem Geschirr vorgesehenen Körbe, dem Einstellen von Geschirr während der Öffnungszeit der Tür des Reinigungsgerätes verursacht ist, kann indikativ für die Menge an Geschirr sein, die während des Beladens in den Innenraum des Reinigungsgerätes eingeführt wurde. Erfasst der mindestens eine Reinigungssensor nach einem Beladen eine Änderung hinsichtlich einer Geruchsbelastung im Innenraum des Reinigungsgerätes, wird eine entsprechende Beduftungs- und/oder Desodorierungsinformation erzeugt und es erfolgt ein Auslösen einer Beduftung und/oder Desodorierung. Entsprechend kann die Entwicklung einer Geruchsbelastung, die insbesondere von einem Menschen wahrnehmbar ist, minimiert bzw. verhindert werden. In einer beispielhaften Ausführungsform ist das Auslösen einer Beduftung und/oder Desodorierung während eines Beladens des Reinigungsgerätes, also z.B. während eine Tür des Reinigungsgerätes geöffnet ist, unterbunden. Das Beladen des Reinigungsgerätes ist beispielsweise über von dem mindestens einen Lichtsensor erfassten zweiten Informationen bestimmbar.

Der mindestens eine Temperatursensor ist beispielsweise wenigstens ein Sensor, der zur Erfassung einer Temperatur geeignet ist.

Gemäß einer beispielhaften Ausführungsform aller Aspekte umfasst das Verfahren:
- Adaptieren des vordefinierten Schwellwertes basierend auf den erfassten zweiten Informationen, insbesondere ein Adaptieren des vordefinierten Schwellwertes mittels eines Nullpunkt-Kalibrierens im Fall, dass die Temperatur einen vordefinierten Temperaturschwellwert unterschritten und der Lichteintritt ein Ausräumen des Reinigungsgerätes signalisiert hat.

Mittels des Adaptieren ist insbesondere ein Kalibrieren des mindestens einen Geruchssensors möglich. Bei der Kalibrierung wird beispielsweise der zum Vergleich mit den erfassten ersten Informationen herangezogene vordefinierte Schwellwert festgelegt. Eine sogenannte Nullpunkt-Kalibrierung kann beispielsweise im Leer- oder Sauberzustand des Reinigungsgerätes durchgeführt werden und kann insbesondere (automatisch) bei Erkennung des Leer- oder Sauberzustand des Reinigungsgerätes gestartet bzw. getriggert werden, z.B. durch ein entsprechendes Steuersignal des Prozessors.

Um zu erkennen, ob das Reinigungsgerät in einem Leer- oder Sauberzustand ist, wird zunächst überprüft, ob die Temperatur im Innenraum des Reinigungsgerätes unter einem vordefinierten Temperaturschwellwert liegt. Da bei der Durchführung eines Reinigungszyklus des Reinigungsgerätes regelmäßig eine erhöhte Temperatur im Innenraum des Reinigungsgerätes herrscht, liegt also für den Fall, dass die Temperatur gemäß den erfassten zweiten Informationen über dem vordefinierten Temperaturschwellwert liegt, kein Leer- oder Sauberzustand des Reinigungsgerätes vor.

Für den Fall, dass die Temperatur unter dem vordefinierten Schwellwert liegt, wird beispielsweise zusätzlich überprüft, ob ein Lichteintritt (z.B. von mindestens einem Lichtsensor) erfasst wurde. Über den Lichteintritt in Kombination mit einem Temperaturabfall, der z.B. über mindestens einen Temperatursensor erfassbar ist, kann der Lichteintritt signalisieren, dass ein Ausräumen (z.B. von Geschirr) des Reinigungsgerätes stattgefunden hat. Für den Fall, dass kein Lichteintritt erfasst wurde und entsprechend kein ausgeräumtes Reinigungsgerät signalisiert wird, liegt entsprechend liegt kein Leer- oder Sauberzustand des Reinigungsgerätes vor.

Wenn die Temperatur im Innenraum des Reinigungsgerätes unter einen bestimmten Temperaturschwellwert gefallen ist, und das Reinigungsgerät ausgeräumt wurde, erfolgt beispielsweise das Adaptieren des vordefinierten Schwellwertes, so dass z.B. eine Nullpunkt-Kalibrierung durchgeführt wird. Hierbei werden die von dem mindestens einen Geruchssensor erfassten ersten Informationen (z.B. absolute von dem mindestens einen Geruchssensor erfasste Messwerte) gespeichert. Diese gespeicherten ersten Informationen dienen beispielsweise der Alterungsüberwachung des mindestens einen Geruchssensors. Die Auswertung dieser gespeicherten ersten Informationen kann beispielsweise dazu genutzt werden, um einen Nutzer des Reinigungsgerätes zu informieren, wann die Empfindlichkeit des mindestens einen Geruchssensors abnimmt und ein eventueller Austausch von diesem erforderlich ist, um eine ordnungsgemäße Funktionsweise der (automatischen) Beduftung und/oder Desodorierung sicherstellen zu können.

Die von dem mindestens einen Geruchssensor erfassten ersten Informationen (z.B. absolute von dem mindestens einen Geruchssensor erfasste Messwerte), die im Leer- oder Sauberzustand des Reinigungsgerätes gespeichert werden, charakterisieren einen Zustand des Innenraums des Reinigungsgerätes, der annähernd frei von Störgerüchen ist, also eine neutrale Geruchsbelastung im Innenraum des Reinigungsgerätes. Um zu überprüfen, ob eine neutrale Geruchsbelastung im Innenraum des Reinigungsgerätes vorliegt, kann beispielsweise das Auslösen der Freisetzung von einer definierten Menge von Beduftungs- und/oder Desodorierungsmittel durchgeführt werden. Hierzu muss sichergestellt sein, dass zumindest eins der freigesetzten Beduftungs- und/oder Desodorierungsmittel zu einer Signalvollauslenkung (100%) des mindestens einen Geruchssensors führt, jedoch nicht zu einer Übersättigung. Für den Fall, dass der mindestens eine Geruchssensor als Ethanolsensor ausgebildet ist, kann beispielsweise als Beduftungs- und/oder Desodorierungsmittel Ethanol oder ein vergleichbar reagierender Alkohol freigesetzt werden. Ist dies nicht möglich, kann alternativ beispielsweise eine Kalibriersubstanz oder eine entsprechende Mischung, die separat bevorratet werden (z.B. im Dosiergerät) in den Innenraum des Reinigungsgerätes eingebracht werden, oder alternativ kann die Kalibriersubstanz oder die entsprechende Mischung von außen zugeführt werden.

Sollte keine Signalvollauslenkung (100%) von dem mindestens einen Geruchssensors erfasst werden, kann eine entsprechende Adaption des Schwellwertes vorgenommen werden, so dass trotz beispielsweise durch Alterung beeinträchtigte Funktion des mindestens einen Geruchssensors weiterhin die Erfassung einer Geruchsbelastung im Innenraum des Reinigungsgerätes mittels des mindestens einen Geruchssensors möglich ist.

Das Adaptieren des vordefinierten Schwellwertes ist alternativ oder zusätzlich von einem Nutzer manuell durchführbar. Der Nutzer kann beispielsweise anhand des Adaptierens des vordefinierten Schwellwertes eine für ihn maximal erträgliche unangenehme Geruchsbelastung einstellen. Weiterhin kann der Nutzer beispielsweise eine (manuelle) Anpassung der Menge an im Rahmen des Auslösens einer Beduftung und/oder Desodorierung freigesetzte Beduftungs- und/oder Desodorierungsmittel einstellen, z.B. mittels eines Mengenschwellwertes. Der Mengenschwellwert kann beispielsweise als Faktor gewichtet in die erzeugte Beduftungs- und/oder Desodorierungsinformation einfließen. Beispielsweise kann ein Nutzer eine verstärkte Beduftung und/oder Desodorierung wünschen, wenn der Nutzer z.B. sehr empfindlich für eine unangenehme Geruchsbelastung ist. Insbesondere kann ein Einstellen eines Mengenschwellwertes sinnvoll sein, wenn die unangenehme Geruchsbelastung im Innenraum des Reinigungsgerätes durch Substanzen hervorgerufen wird, die beispielsweise der mindestens eine Geruchssensor nicht erfassen kann.

Eine beispielhafte Ausgestaltung nach allen Aspekten sieht vor, dass der mindestens eine Geruchssensor und der mindestens eine Lichtsensor und/oder der mindestens eine Temperatursensor ein Sensorarray ausbilden.

Das Sensorarray umfasst beispielsweise mindestens einen Geruchssensor und mindestens einen Lichtsensor. Alternativ oder zusätzlich umfasst das Sensorarray mindestens einen Temperatursensor. Das Sensorarray umfasst beispielsweise mehrere Geruchssensoren und einen Lichtsensor. Das Sensorarray umfasst beispielsweise eine Vielzahl von einzelnen Sensoren oder ein weiteres Sensorarray, auf dem wiederrum verschiedene Sensoren anordenbar sind. Die jeweiligen Sensoren können modulartig ausgebildet sein, so dass diese auf dem Sensorarray austauschbar sind, z.B. als Steckmodul. Alternativ oder zusätzlich ist beispielsweise das Sensorarray austauschbar, z.B. ist das Sensorarray als tauschbares Modul ausgebildet. Insbesondere umfasst das Sensorarray mehrere Sensoren, mittels der verschiedenste organische Gruppen erfassbar sind, wie z.B. Alkohole, Ketone, Thiole, Mercaptane, Amine, Ester, Kohlenwasserstoffe, oder eine Kombination hiervon.

Gemäß einer beispielhaften Ausführungsform aller Aspekte wird das Verfahren zwischen zwei Reinigungszyklen des Reinigungsgerätes durchgeführt und/oder gesteuert.

Gemäß einer beispielhaften Ausführungsform aller Aspekte wird das Auslösen einer Beduftung und/oder Desodorierung durchgeführt, wenn die erzeugte Beduftungs- und/oder Desodorierungsinformation indikativ für eine von einem Nutzer als unangenehm empfundene Geruchsbelastung im Innenraum des Reinigungsgerätes ist. Hierzu kann beispielsweise der vordefinierte Schwellwert entsprechend adaptiert werden, z.B. durch eine Nutzereingabe, die insbesondere indikativ für das individuelle Nutzerempfinden hinsichtlich einer (Geruchs-) Schwelle ist, die von dem Nutzer als unangenehm empfunden wird. Geruchseindrücke sind sehr subjektive und komplexe Wahrnehmungen und werden von Menschen äußerst unterschiedlich empfunden. Deshalb ist es gerade in diesem Gebiet besonders effektiv, mit cognitiven, selbstlernenden und/oder adaptiven Verfahren zu arbeiten, um der Vielzahl an hedonischen Dimensionen von Geruch beschreiben und bewerten und letztlich dem einzelnen Nutzer eine auf ihn abgestimmte Lösung anbieten zu können.

Eine Ausführungsform aller Aspekte sieht vor, dass das Auslösen der Beduftung und/oder Desodorierung unterschiedliche Beduftungs- und/oder Desodorierungsmittel, insbesondere in Abhängigkeit der Art der Geruchsbelastung im Innenraum des Reinigungsgerätes, freisetzt. Entsprechend der (z.B. erfassten und/oder bestimmten) Art der Geruchsbelastung im Innenraum des Reinigungsgerätes kann eine Beduftung und/oder Desodorierung erfolgen. Eine Möglichkeit, um die Art einer Geruchsbelastung zu beschreiben, ergibt sich beispielsweise aus einer sogenannten Kohonen-Karte. Eine derartige Kohonen-Karte, z.B. von Mamlouk et al aus dem Jahr 2003, stellt 32 Geruchsdimensionen dar. Die Geruchsdimensionen beschreiben mit abstrakten Begriffen, wie z.B. "fischig" oder "Plastik" einen olfaktorischen Raum, hinter dem sich eine Vielzahl von Stoffen, die jeweils diese Geruchsdimension hervorrufen, verbirgt. Stellt beispielsweise ein Geruchssensor ein Amin fest, kann mit Hilfe einer derartigen Kohonen-Karte das von dem Geruchssensor erzeugte elektrische Signal in eine Geruchsbeschreibung übersetzt werden. Entsprechend der Geruchsdimension kann auch das passende Beduftungs- und/oder Desodorierungsmittel gewählt werden. Alternativ oder zusätzlich lässt sich mittels der Geruchsdimension einer Kohonen-Karte feststellen, ob eine Geruchsbelastung (überhaupt) vorliegt. Wenn ein Stoff mittels des Geruchssensors erfasst wird, der in die olfaktorische Kategorie Schlechtgeruch fällt (im Gegensatz zu Geruchsdimensionen, die nicht in die olfaktorische Kategorie Schlechtgeruch fallen), kann dies verwendet werden, um eine entsprechende Beduftung und/oder Desodorierung auszulösen.

Gemäß einer beispielhaften Ausführungsform aller Aspekte setzt das Auslösen der Beduftung und/oder Desodorierung unterschiedliche Beduftungs- und/oder Desodorierungsmittel frei, insbesondere in Abhängigkeit der Art der Geruchsbelastung im Innenraum des Reinigungsgerätes. Alternativ oder zusätzlich setzt das Auslösen der Beduftung und/oder Desodorierung unterschiedliche Mengen des Beduftungs- und/oder Desodorierungsmittels frei, insbesondere in Abhängigkeit der Intensität der Geruchsbelastung im Innenraum des Reinigungsgerätes.

Eine beispielhafte Ausgestaltung nach allen Aspekten sieht vor, dass das Beduftungs- und/oder Desodorierungsmittel oder die Beduftungs- und/oder Desodorierungsmittel derart eingerichtet und/oder ausgebildet sind, dass der mindestens eine Geruchssensor das Beduftungs- und/oder Desodorierungsmittel oder die Beduftungs- und/oder Desodorierungsmittel nicht erfassen kann. Das Beduftungs- und/oder Desodorierungsmittel oder die Beduftungs- und/oder Desodorierungsmittel umfassen oder beinhalten entsprechend keinen Stoff, auf den der mindestens eine Geruchssensor reagiert. Der mindestens eine Geruchssensor ist also in diesem Fall "blind" für das Beduftungs- und/oder Desodorierungsmittel oder die Beduftungs- und/oder Desodorierungsmittel. Entsprechend kann der mindestens eine Geruchssensor ausschließlich Stoffe bzw. eine Geruchsbelastung feststellen, die durch eine Geruchsbelastung im Innenraum des Reinigungsgerätes (z.B. durch verunreinigtes Geschirr) hervorgerufen werden. Im Rahmen des Erzeugens einer Beduftungs- und/oder Desodorierungsinformation muss entsprechend bereits freigesetztes Beduftungs- und/oder Desodorierungsmittel nicht berücksichtigt werden. In diesem Fall ist für ein Adaptieren des vordefinierten Schwellwertes, beispielsweise im Rahmen der Nullpunkt-Kalibrierung, ein separat zu dem Beduftungs- und/oder Desodorierungsmittel zu bevorratendes Kalibrierungsmittel erforderlich oder dieses muss von außen (z.B. manuell durch einen Nutzer) zugeführt werden.

Die Aufgabe wird gegenständlich durch ein beispielhaftes Dosiergerät zur Positionierung im Innenraum eines Reinigungsgerätes nach einem zweiten Aspekt gelöst, das Dosiergerät umfassend:
- mindestens eine Kartusche zur Bevorratung von mindestens einer Zubereitung eines Beduftungs- und/oder Desodorierungsmittels;
- eine Sensoreinheit umfassend mindestens einen Geruchssensor;
- eine Steuereinheit, wobei mittels der Steuereinheit eine Beduftungs- und/oder Desodorierungsinformation basierend auf von der Sensoreinheit erfassten ersten Informationen erzeugbar ist;
- einen Aktuator zum Auslösen einer Beduftung und/oder Desodorierung basierend auf der erzeugten Beduftungs- und/oder Desodorierungsinformation, wobei mindestens eine Zubereitung eines Beduftungs- und/oder Desodorierungsmittels freisetzbar ist.

In einer beispielhaften Ausführungsform gemäß aller Aspekte umfasst die Sensoreinheit mindestens einen Lichtsensor und/oder mindestens einen Temperatursensor. Die Sensoreinheit umfasst beispielsweise mindestens einen Geruchssensor und mindestens einen Lichtsensor. Alternativ oder zusätzlich umfasst die Sensoreinheit mindestens einen Temperatursensor. Die Sensoreinheit umfasst beispielsweise mehrere Geruchssensoren und einen Lichtsensor. Die Sensoreinheit umfasst beispielsweise eine Vielzahl von einzelnen Sensoren oder ein Sensorarray, auf dem verschiedene Sensoren anordenbar sind. Die jeweiligen Sensoren können modulartig ausgebildet sein, so dass diese auf der Sensoreinheit austauschbar sind, z.B.als Steckmodul. Alternativ oder zusätzlich ist beispielsweise das Sensorarray austauschbar, z.B. ist das Sensorarray als tauschbares Modul ausgebildet. Insbesondere umfasst die Sensoreinheit mehrere Sensoren, mittels derer verschiedenste organische Gruppen erfassbar sind, wie z.B. Alkohole, Ketone, Thiole, Mercaptane, Amine, Ester, Kohlenwasserstoffe, oder eine Kombination hiervon.

Von dem Dosiergerät sind die zum Betrieb notwendige Steuereinheit, Sensoreinheit sowie optional wenigstens eine Energiequelle umfasst. In einer weiteren Ausgestaltung umfasst das Dosiergerät wenigstens einen Aktuator, der derart mit wenigstens einer Energiequelle und der Steuereinheit verbunden ist, dass ein Steuersignal der Steuereinheit eine Bewegung des Aktuators bewirkt.

In einer beispielhaften Ausgestaltung kann das Dosiergerät aus einem spritzwassergeschütztem Gehäuse gebildet sein, dass das Eindringen von Spritzwasser, wie es beispielsweise bei der Verwendung in einer Geschirrspülmaschine auftreten kann, in das Innere des Dosiergeräts verhindert.

Eine beispielhafte Ausgestaltung sieht vor, insbesondere die Energiequelle, die Steuereinheit sowie die Sensoreinheit derart zu vergießen, dass das Dosiergerät im Wesentlichen wasserdicht, das Dosiergerät also auch bei vollständigem Umschluss mit Flüssigkeit funktionsfähig ist. Als Vergussmaterialien können beispielsweise mehrkomponentige Epoxyd-, und Acrylat-Vergußmassen wie Methacrylatester, Urethan-Metha und Cyanacrylate oder Zweikomponenten-Materialien mit Polyurethanen, Silikonen, Epoxydharzen verwendet werden.

Eine Alternative oder Ergänzung zum Vergießen stellt das Verkapseln der Bauteile in einem entsprechend ausgestalteten, feuchtigkeitsdichten Gehäuse dar. Eine derartige Ausgestaltung wird an nachfolgender Stelle noch näher erläutert.

In einer beispielhaften Ausgestaltung umfasst das Dosiergerät wenigstens eine erste Schnittstelle, welche in oder an einem wasserführendem Gerät wie insbesondere ein wasserführendes Haushaltsgerät, bevorzugt eine Geschirrspülmaschine ausgebildeten korrespondierenden Schnittstelle in derart zusammenwirkt, dass eine Übertragung von elektrischer Energie von dem wasserführenden Gerät zum Dosiergerät verwirklicht ist.

In einer Ausgestaltung ist die wenigstens eine Schnittstelle durch Steckverbinder ausgebildet. In einer weiteren Ausgestaltung kann die wenigstens eine Schnittstelle in derart ausgebildet sein, dass eine drahtlose Übertragung von elektrischer Energie bewirkt ist, zum Beispiel mittels Induktion.

Hierbei ist es insbesondere bevorzugt, dass die Schnittstellen induktive Sender bzw. Empfänger elektromagnetischer Wellen sind. So kann insbesondere die Schnittstelle eines wasserführenden Geräts, wie etwa einer Geschirrspülmaschine, als eine mit Wechselstrom betriebene Sender-Spule mit Eisenkern und die Schnittstelle des Dosiergeräts als eine Empfänger-Spule mit Eisenkern ausgebildet sein.

In einer Ausführungsform kann die Energiequelle auch in zumindest einer Kartusche angeordnet sein. Dann kann die Kartusche mit dem Dosiergerät elektrisch gekoppelt sein. Da die Kartusche als Austauschware vorzugsweise in Abständen ohnehin ausgetauscht wird, kann somit eine Energieversorgung für das Dosiergerät gewährleistet werden.

In einer beispielhaften Weiterentwicklung ist jeweils eine zweite Schnittstelle am Dosiergerät und dem wasserführenden Gerät, wie etwa einer Geschirrspülmaschine, zur Übertragung von elektromagnetischen Signalen, welche insbesondere Betriebszustands-, Mess- und/oder Steuerinformationen des Dosiergeräts und/oder des wasserführenden Geräts wie einer Geschirrspülmaschine repräsentieren, ausgebildet.

Insbesondere kann eine derartige Schnittstelle derart ausgebildet sein, dass eine drahtlose Übertragung von elektromagnetischen Signalen bewirkt ist. Die drahtlose Übertragung von Daten kann beispielsweise mittels Funkübertragung oder IR-Übertragung realisiert sein.

Unter einer Kartusche im Sinne dieser Anmeldung kann ein Packmittel verstanden werden, das dazu geeignet ist, fließfähige oder streufähige Substanzen wie Beduftungs- und/oder Desodorierungsmittel zu umhüllen oder zusammenzuhalten und das zur Abgabe der Substanz vorzugsweise an ein Dosiergerät koppelbar ist. Die in der Kartusche aufnehmbare Substanz ist für eine wiederholte Dosierung vorgesehen. Das Dosiergerät umfasst vorzugsweise mindestens zwei Kartuschen, die jeweils mit dem Dosiergerät koppelbar sind. Es kann zumindest eine, vorzugsweise jede der Kartuschen lösbar mit dem Dosiergerät gekoppelt sein. Für die Beaufschlagung eines Volumens von 100 bis 200 I mit Beduftung und/oder Desodorierung (z.B. zur Beseitigung einer unangenehmen Geruchsbelastung im Innenraum eines Reinigungsgerätes) werden nur geringe Mengen an aktiven Stoffen (z.B. Beduftungs- und/oder Desodorierungsmittel) benötigt, da viele Riechstoffe wie beispielsweise Beduftungs- und/oder Desodorierungsmittel sehr niedrige Geruchsschwellenwerte aufweisen. Im Folgenden sind einige Beispiele für Beduftungs- und/oder Desodorierungsmittel und deren Geruchsschwellenwerten in Wasser aufgeführt:

**Geruchsschwelle in Wasser:**

| | |
|---|---|
| Linalool: | 6 µg/ml |
| Citronellol: | 10 µg/ml |
| Beta-Jonon: | 0,007 µg/ml |
| Beta-Damascenon: | 0,002 µg/ml |

Hieraus ergibt sich, dass Beduftungs- und/oder Desodorierungsmittel schon in kleinen Mengen wirksam sein können. Das bedeutet auch, dass nur kleine Volumina von Beduftungs- und/oder Desodorierungsmittel bevorratet werden müssen.

In einer beispielhaften Ausgestaltung können die mindestens zwei Kartuschen mit einer Mehrzahl von räumlich voneinander separierten Kammern jeweils zur Aufnahme voneinander verschiedener Substanzen eines Beduftungs- und/oder Desodorierungsmittels ausgebildet sein. Insbesondere kann eine Kartusche mehrere Kammern umfassen, die mit voneinander verschiedenen Beduftungs- und/oder Desodorierungsmitteln befüllbar sind. Derart ist ein kombinierter Einsatz von Beduftungs- und/oder Desodorierungsmitteln ermöglicht.

In einer beispielhaften Ausgestaltung weist die Kartusche wenigstens eine Auslassöffnung auf, die derart angeordnet ist, dass eine schwerkraftbewirkte Substanzfreisetzung aus dem Behälter in der Gebrauchsstellung des Dosiergeräts bewirkt werden kann. Hierdurch werden keine weiteren Fördermittel zur Freisetzung von Substanz aus dem Behälter benötigt, wodurch der Aufbau des Dosiergeräts einfach und die Herstellungskosten niedrig gehalten werden können.

In einer weiteren beispielhaften Ausgestaltungsform kann wenigstens eine zweite Kammer zur Aufnahme wenigstens einer zweiten fließ oder streufähigen Substanz vorgesehen sein, wobei die zweite Kammer wenigstens eine Auslassöffnung aufweist, die derart angeordnet ist, dass eine schwerkraftbewirkte Produktfreisetzung aus der zweiten Kammer in der Gebrauchsstellung des Dosiergeräts bewirkt ist. Die Anordnung einer zweiten Kammer ist insbesondere dann vorteilhaft, wenn in den voneinander getrennten Behältern Substanzen bevorratet sind, die üblicherweise nicht miteinander lagerstabil sind.

Des Weiteren ist es vorstellbar, dass mehr als zwei, insbesondere drei bis vier Kammern in bzw. an einer Kartusche vorgesehen sind. Insbesondere kann eine der Kammern zur Abgabe von flüchtigen Substanzen wie etwa eines Duftstoffs an die Umgebung ausgestaltet sein.

In einer weiteren beispielhaften Ausgestaltung kann die Kartusche einstückig ausgebildet sein. Hierdurch lässt sich die Kartusche, insbesondere durch geeignete Blasformverfahren, kostengünstig in einem Herstellungsschritt ausbilden. Die Kammern der Kartusche können hierbei beispielsweise durch Stege oder Materialbrücken voneinander separiert sein.

Die Kartusche kann auch mehrstückig durch im Spritzguss hergestellte und anschließend zusammengefügte Bauteile gebildet sein. Ferner ist es denkbar, dass die Kartusche derart mehrstückig ausgeformt ist, dass wenigstens eine Kammer, vorzugsweise alle Kammern, einzeln aus dem Dosiergerät entnehmbar oder in das Dosiergerät einsetzbar sind. Hierdurch ist es möglich, bei einem unterschiedlich starken Verbrauch einer Substanz aus einer Kammer, eine bereits entleerte Kammer auszutauschen, während die übrigen, die noch mit einer Substanz befüllt sein können, in dem Dosiergerät verbleiben. Somit kann ein gezieltes und bedarfsgerechtes Nachfüllen der einzelnen Kammern bzw. deren Substanzen erreicht werden.

Die Kammern einer Kartusche können durch geeignete Verbindungsmethoden aneinander fixiert sein, so dass eine Behältereinheit gebildet ist. Die Kammern können durch eine geeignete formschlüssige, kraftschlüssige oder stoffschlüssige Verbindung lösbar oder unlösbar gegeneinander fixiert sein. Insbesondere kann die Fixierung durch eine oder mehrere der Verbindungsarten aus der Gruppe der Snap-In Verbindungen, Klettverbindungen, Pressverbindungen, Schmelzverbindungen, Klebverbindungen, Schweißverbindungen, Lötverbindungen, Schraubverbindungen, Keilverbindungen, Klemmverbindungen oder Prellverbindungen erfolgen. Insbesondere kann die Fixierung auch durch einen Schrumpfschlauch (sog. Sleeve) ausgebildet sein, der in einem erwärmten Zustand über die gesamte oder Abschnitte der Kartusche gezogen wird und die Kammern bzw. die Kartusche im abgekühlten Zustand fest umschließt.

Um vorteilhafte Restentleerungseigenschaften der Kammern bereitzustellen, kann der Boden der Kammern trichterförmig zur Abgabeöffnung hin geneigt sein. Des Weiteren kann die Innenwand einer Kammer durch geeignete Materialwahl und/oder Oberflächenausgestaltung derart ausgebildet sein, dass eine geringe Materialanhaftung der Substanz an der inneren Kammerwand realisiert ist. Auch durch diese Maßnahme lässt sich die Restentleerbarkeit einer Kammer weiter optimieren.

Die Kammern einer Kartusche können gleiche oder voneinander verschiedene Füllvolumina aufweisen. Bei einer Konfiguration mit zwei Kammern beträgt das Verhältnis der Behältervolumina bevorzugt 5:1, bei einer Konfiguration mit drei Kammern bevorzugt 4:1:1, wobei diese Konfigurationen insbesondere zur Verwendung in Geschirrspülmaschinen geeignet sind.

In oder an einer Kammer kann eine Dosierkammer, in Fließrichtung der Substanz vor der Auslassöffnung ausgebildet sein. Durch die Dosierkammer wird die Substanzmenge, die bei der Freisetzung von Substanz aus der Kammer an die Umgebung abgegeben werden soll, festgelegt. Dies ist insbesondere dann vorteilhaft, wenn das Verschlusselement des Dosiergeräts, das die Substanzabgabe aus einer Kammer an die Umgebung bewirkt, nur in einen Abgabe und einen Verschlusszustand ohne Kontrolle der Abgabemenge versetzt werden kann. Durch die Dosierkammer wird dann gewährleistet, dass ohne eine unmittelbare Rückkopplung der abgegebenen Substanzmenge eine vordefinierte Menge an Substanz freigesetzt wird. Die Dosierkammern können einstückig oder mehrstückig ausgeformt sein.

Gemäß einer weiteren beispielhaften Weiterentwicklung können eine oder mehrere Kammern neben einer Auslassöffnung jeweils eine flüssigkeitsdicht verschließbare Kammeröffnung aufweisen. Durch diese Kammeröffnung ist es beispielsweise ermöglicht, in dieser Kammer aufbewahrte Substanz nachzufüllen.

Zur Belüftung der Kammern können insbesondere im Kopfbereich der Kammer Belüftungsmöglichkeiten vorgesehen sein, um einen Druckausgleich bei fallendem Befüllstand der Kammern zwischen dem Inneren der Kammern und der Umgebung zu gewährleisten. Diese Belüftungsmöglichkeiten können beispielsweise als Ventil, insbesondere Silikonventil, Micro-Öffnungen in der Kammerwand oder dergleichen ausgebildet sein.

Sollte gemäß einer weiteren Ausgestaltung nicht die Kammer direkt belüftet werden, sondern über das Dosiergerät oder keine Belüftung, zum Beispiel bei der Verwendung flexibler Behältnisse, wie beispielsweise Beutel, vorgesehen sein, so hat dies den Vorteil, dass bei erhöhten Temperaturen im Laufe eines Spülzyklus einer Reinigungsmaschine durch die Erwärmung des Kammerinhalts ein Druck aufgebaut wird, der die zu dosierenden Substanzen in Richtung der Auslassöffnungen drückt, so dass hierdurch eine gute Restentleerbarkeit der Kartusche erreichbar ist. Ferner besteht bei einer derartigen, vorzugsweise luftfreien Verpackung nicht die Gefahr einer Oxidation der Substanzen, weshalb eine Beutelverpackung oder auch Bag-In-Bottle-Verpackung insbesondere für oxidationsempfindliche Substanzen zweckmäßig sein kann.

Die Kartusche weist üblicherweise ein Füllvolumen von < 5.000 ml, insbesondere < 1.000 ml, bevorzugt < 500 ml, besonders bevorzugt < 250 ml, ganz besonders bevorzugt < 50 ml auf.

Die Kartusche kann jede beliebige Raumform annehmen. Sie kann beispielsweise würfelartig, kugelförmig oder plattenartig ausgebildet sein.

Die Kartusche und das Dosiergerät können insbesondere derart bezüglich ihrer Raumform ausgestaltet sein, dass sie einen möglichst geringen Nutzvolumenverlust insbesondere in einer Geschirrspülmaschine gewährleisten.

In einer beispielhaften Ausgestaltung weist zumindest eine Kartusche eine Erschöpfungsanzeige auf. Dies kann eine sogenannte End-of-Life Signalisierung sein, bei der anzeigbar ist, dass das in einer Kartusche oder einer Kammer einer Kartusche aufgenommen Beduftungs- und/oder Desodorierungsmittel erschöpft bzw. nahezu erschöpft ist. Um eine unmittelbare optische Füllstandskontrolle bereitzustellen, kann die Kartusche zumindest abschnittsweise aus einem transparenten Material geformt sein. Ferner kann eine in der Kartusche befindliche Restmenge über eine End-of-life Signalisierung realisiert sein. Hierbei kann beispielsweise über das Wissen des in der Kartusche aufgenommenen Volumens von Beduftungs- und/oder Desodorierungsmittel und über die Menge, die pro ausgeführter und/oder gesteuerter Dosierung von Beduftungs- und/oder Desodorierungsmittel abgegeben wird, eine Verrechnung erfolgen, so dass die Restmenge innerhalb der Kartusche von Beduftungs- und/oder Desodorierungsmittel berechnet werden kann.

Um hitzeempfindliche Bestandteile einer in einer Kartusche befindlichen Substanz vor Wärmeeinwirkung zu schützen, kann die Kartusche aus einem Material mit einer geringen Wärmeleitfähigkeit hergestellt sein.

Eine weitere Möglichkeit zur Verminderung des Hitzeeinflusses auf eine Substanz in der Kartusche ist es, die Kartusche durch geeignete Maßnahmen zu isolieren, zum Beispiel durch die Verwendung von Wärmedämmmaterialien wie etwa Styropor, die die Kartusche oder eine Kammer der Kartusche in geeigneter Weise ganz oder teilweise umschließen.

Eine weitere Maßnahme zum Schutz hitzeempfindlicher Substanzen in einer Kartusche ist, bei einer Mehrzahl von Kammern, deren Anordnung zueinander. So ist es beispielsweise denkbar, dass die Kammer, die ein hitzeempfindliches Produkt beinhaltet, teilweise oder vollständig von wenigstens einer weiteren, mit einer Substanz befüllten Kammer umschlossen ist, wobei diese Substanz und diese Kammer in dieser Konfiguration als Wärmeisolation für die umschlossene Kammer fungieren. Dies bedeutet, dass eine erste Kammer, die eine hitzeempfindliche Substanz beinhaltet, teilweise oder vollständig von wenigstens einer weiteren, mit einer Substanz befüllten Kammer umschlossen ist, so dass die hitzeempfindliche Substanz in der ersten Kammer bei Erwärmung der Umgebung einen langsameren Temperaturanstieg aufweist, als die Substanz in den umgebenden Kammern.

Um eine weitere Verbesserung der Wärmeisolation herbeizuführen, können bei der Verwendung von mehr als zwei Kammern, die Kammern nach dem Matroschka-Prinzip umeinander angeordnet werden, so dass eine mehrschichtige Isolationsschicht gebildet ist.

Insbesondere ist es vorteilhaft, dass wenigstens eine Substanz, die in einer umschließenden Kammer bevorratet ist, eine Wärmeleitfähigkeit zwischen 0,01 und 5 W/m.K, bevorzugt zwischen 0,02 und 2 W/m.k, insbesondere bevorzugt zwischen 0,024 und 1 W/m.K aufweist.

Die Kartusche kann insbesondere formstabil ausgebildet sein. Es ist jedoch auch denkbar, die Kartusche als flexibles Packmittel wie etwa als Tube auszugestalten. Des Weiteren ist es auch möglich, flexible Behältnisse wie Beutel zu verwenden, insbesondere, wenn sie gemäß des "bag-in-bottle"-Prinzips in ein im Wesentlichen formstabiles Aufnahmebehältnis eingesetzt werden. Durch die Verwendung flexibler Packmittel entfällt - anders als bei den eingangs beschriebenen formstabilen Packmitteln (Kartusche) - die Notwendigkeit ein Belüftungssystem zum Druckausgleich vorzusehen.

In einer beispielhaften Ausführungsform kann die Kartusche ein RFID-Etikett aufweisen, dass zumindest Informationen über den Inhalt der Kartusche beinhaltet und das durch die Sensoreinheit berührungslos auslesbar ist.

Diese Informationen können beispielsweise verwendet werden, um ein in der Steuereinheit gespeichertes Dosierprogramm, welches zum Beispiel eine Beduftung und/oder Desodorierung auslöst, auszuwählen. Hierdurch kann sichergestellt werden, dass stets ein für ein bestimmtes Beduftungs- und/oder Desodorierungsmittel optimales Dosierprogramm verwendet wird. Es kann auch vorgesehen sein, dass bei nicht Vorhandensein eines RFID-Labels oder bei einem RFID-Label mit einer falschen oder fehlerhaften Kennung, keine Dosierung durch das Dosiergerät erfolgt und statt dessen ein optisches oder akustisches Signal erzeugt wird, dass den Benutzer auf den vorliegenden Fehler hinweist.

Um einen Fehlgebrauch einer jeweiligen Kartusche auszuschließen, können die Kartuschen auch strukturelle Elemente aufweisen, die mit korrespondierenden Elementen des Dosiergeräts nach dem Schlüssel-Schloss-Prinzip zusammenwirken, so dass beispielsweise nur Kartuschen eines bestimmten Typs an das Dosiergerät koppelbar sind. Ferner ist es durch diese Ausgestaltung möglich, dass Informationen über die an das Dosiergerät gekoppelten Kartusche an die Steuereinheit übertragen werden.

Die Auslassöffnungen einer Kartusche können auf einer Linie angeordnet, wodurch eine schlanke, tellerförmige Ausbildung des Dosiergeräts ermöglicht ist.

Die Kartusche kann beispielsweise insbesondere zur Aufnahme von (zum Beispiel fließfähigen) Beduftungs- und/oder Desodorierungsmittel ausgebildet sein. Besonders bevorzugt weist eine derartige Kartusche eine Mehrzahl von Kammern zur räumlich separierten Aufnahme jeweils voneinander verschiedener Substanzen eines Beduftungs- und/oder Desodorierungsmittels.

Die Kartusche kann einen Kartuschenboden umfassen, der in Gebrauchsstellung in Schwerkraftrichtung nach unten gerichtet ist und bei dem wenigstens zwei Kammern jeweils mindestens eine am Kartuschenboden angeordnete Auslassöffnung vorgesehen ist.

Ferner kann die Kartusche aus wenigstens zwei miteinander stoffschlüssig verbundenen Elementen gebildet sein, wobei die Verbindungskante der Elemente am Kartuschenboden außerhalb der Auslassöffnungen verläuft, die Verbindungskante die Auslassöffnungen also nicht schneidet.

Die stoffschlüssige Verbindung kann beispielsweise durch Kleben, Schweißen, Löten, Pressen oder Vulkanisieren hergestellt sein.

In einer beispielhaften Ausgestaltung verläuft die Verbindungskante entlang der Kopf-, Boden- und Seitenflächen der Kartusche. Hierdurch können zwei Kartuschenelemente insbesondere im Spritzgussverfahren hergestellt werden, wobei entweder beide Elemente wannenförmig ausgebildet sind oder ein Element wannenförmig und das zweite Element deckelartig.

Zur Ausbildung einer Zwei- oder Mehrkammerkartusche kann wenigstens eines der beiden Kartuschenelemente wenigstens einen Trennsteg umfassen, der im zusammengefügten Zustand der Elemente jeweils zwei benachbarte Kammern der Kartusche voneinander trennt.

Alternativ zur Ausbildung einer der Kartuschen durch zwei schalenförmige Kartuschenelemente ist es auch denkbar, dass ein Kartuschenelement als napfartiger Behälter mit wenigstens einer Kammer und das zweite Element der Kartuschenboden oder -kopf ist, der mit dem napfartigen Behälter flüssigkeitsdicht entlang der Verbindungskante verbunden ist.

Selbstverständlich ist es auch denkbar, die oben erwähnten Kartuschenkonfigurationen in beliebig geeigneter Weise miteinander zu kombinieren. Beispielsweise ist es möglich eine Zweikammerkartusche aus einem wannenförmigen und einem deckelartigen Kartuschenelement zu bilden und eine dritte ein- oder mehrstückige Kammer am Kopf oder Mantelfläche der so gebildeten Kartusche anzuordnen.

Insbesondere kann eine derartige, weitere Kammer zur Aufnahme einer Substanz an der jeweiligen Kartusche angeordnet und in derart konfiguriert sein, dass eine Abgabe von flüchtigen Substanzen wie beispielsweise Duftstoffen in die Umgebung der Kammer bewirkt wird.

Gemäß einer beispielhaften Ausführung können die Auslassöffnungen mit jeweils einem Verschluss versehen sein, der im mit einem Dosiergerät gekoppelten Zustand ein Ausfließen von Substanz aus den jeweiligen Kammern erlaubt und im ungekoppelten Zustand der Kartuschen ein Ausfließen von Substanz im Wesentlichen verhindert. Insbesondere ist der Verschluss als Silikonventil ausgestaltet.

Die die jeweilige Kartusche bildenden Kartuschenelemente sind vorzugsweise aus einem Kunststoff gebildet und können in einem gemeinsamen Spritzgussprozess ausgeformt werden, wobei es vorteilhaft sein kann, einen als Scharnier wirkenden Verbindungssteg zwischen den beiden Elementen anzuformen, so dass nach der Ausformung die beiden Elemente durch ein Umklappen aneinander anliegen und stoffschlüssig entlang der Verbindungskante verbunden werden.

In einer weiteren Ausgestaltung kann wenigstens eine Energiequelle, insbesondere eine Batterie oder Akkumulator, an einer oder mehreren der Kartuschen, bevorzugt am Boden einer jeweiligen Kartusche, angeordnet sein. An der Kartusche können des weiteren Mittel zur elektrischen Kopplung der Energiequelle mit dem Dosiergerät vorgesehen sein.

Die Kartusche kann so ausgebildet sein, dass sie lösbar oder fest in oder an dem Dosiergerät, zum Beispiel innerhalb der Geschirrspülmaschine angeordnet werden kann. In einer beispielhaften Ausgestaltung ist jede von mehreren Kartuschen lösbar oder fest mit dem Dosiergerät koppelbar. Derart kann beispielsweise ein Austausch von insbesondere erschöpften, d.h. leeren Kartuschen erfolgen bzw. es kann ein Austausch von Kartuschen erfolgen, von welchen vollständig oder nahezu vollständig die in der Kartusche aufgenommene Substanz verbraucht ist. Es ist beispielsweise ein Austausch von jeder der Kartuschen separat bzw. einzeln möglich. Derart kann nur die verbrauchte Substanz wie Beduftungs- und/oder Desodorierungsmittel ausgetauscht werden.

Eine Steuereinheit im Sinne dieser Anmeldung kann eine Vorrichtung sein, die geeignet ist, das Transportieren von Material, Energie und/oder Information zu beeinflussen und/oder auszuführen und/oder zu steuern. Die Steuereinheit beeinflusst hierzu beispielsweise einen Aktuator mit Hilfe eines Steuersignals. Ein Steuersignal kann Informationen umfassen, insbesondere Messsignale, Parameter oder dergleichen.

In einer beispielhaften Ausgestaltung kann mittels der Steuereinheit eine Mengendosierung von z.B. in einer Kartusche aufnehmbaren Beduftungs- und/oder Desodorierungsmittel durchführbar sein. Die Durchführung einer bedarfsgerechten Dosierfunktion bzw. einer Mengendosierung kann beispielsweise durch eine Abgabe von Beduftungs- und/oder Desodorierungsmittel aus der mit dem Dosiergerät koppelbaren Kartusche sequenziell oder zeitgleich, kontinuierlich oder diskontinuierlich erfolgen. Entsprechend kann die Abgabe von in der Kartuschen aufnehmbaren Beduftungs- und/oder Desodorierungsmittel aktiviert bzw. deaktiviert werden, um entweder eine kontinuierliche oder eine diskontinuierliche Abgabe des Beduftungs- und/oder Desodorierungsmittels durchzuführen. Ferner kann beispielsweise eine zeitgerechte Dosierfunktion durchgeführt werden, zum Beispiel basierend auf einem Steuersignal der Steuereinheit. Derart kann beispielsweise eine Substanz zwischen Reinigungszyklen bzw. Reinigungsgängen einer Geschirrspülmaschine mittels des Dosiergerätes abgegeben werden. Basierend auf einem Steuersignal kann die Beduftung- und/oder Desodorierung im Wesentlichen automatisch und/oder autark erfolgen. Beispielsweise ist keine Eingabe einer Information durch einen Nutzer erforderlich. Aufgrund der von der Steuereinheit erfassten Informationen durch eine oder mehrere Einrichtungen zur Messung und/oder Ermittlung von Informationen, z.B. Sensoren, die im Nachfolgenden näher erläutert werden, kann ein Steuersignal generiert werden, welches eine bedarfsgerechte, d.h. basierend auf den mittels der Sensoreinheit erfassten (ersten) Informationen, Beduftung- und/oder Desodorierung erfolgen bzw. durchgeführt werden bzw. deren Durchführung veranlasst werden.

Eine beispielhafte Ausgestaltung kann vorsehen, dass ein Steuersignal der Steuereinheit das Auslösen einer Aktion bewirkt, insbesondere das Auslösen einer Beduftung und/oder Desodorierung von Beduftungs- und/oder Desodorierungsmitteln bewirkt. Die Aktion ist beispielsweise die Durchführung bzw. die Veranlassung der Durchführung einer vorgenannten Beduftung- und/oder Desodorierung. Denkbar ist auch, dass das Steuersignal eine weitere Aktion bewirkt bzw. deren Durchführung bewirkt. Zum Beispiel kann das Steuersignal an eine weitere, zum Beispiel externe Einrichtung weitergeleitet werden. Die Weiterleitung kann beispielsweise über eine entsprechende Schnittstelle zur Übermittlung von Informationen, insbesondere zur Weiterleitung des Steuersignals ausgebildet sein. Das Steuersignal kann beispielsweise an eine Anzeigeeinrichtung weitergeleitet werden, so dass zum Beispiel Statusinformationen anzeigbar sind, die insbesondere optisch, akustisch und/oder haptisch anzeigbar sind. Derart ist es beispielsweise möglich, das Dosiergerät "von außen" zu beaufsichtigen, zu kontrollieren und/oder zu steuern. Zudem können Prozessinformationen, Kenndaten und/oder von der Sensoreinheit erfassten Messwerte generiert und an eine externe Einrichtung übermittelt werden. Eine externe Einrichtung kann beispielsweise basierend auf dem Steuersignal eine bedarfsgerechte Dosierung unterstützen. Denkbar ist beispielsweise die Verstärkung der Wirkung eines Beduftungs- und/oder Desodorierungsmittels, zum Beispiel einer Desodorierungssubstanz durch die Bestrahlung von Geschirr mittels UV-Strahlung, insbesondere UV-C Strahlung, die basierend auf dem Steuersignal ausgelöst wird. Im Falle einer im Wesentlichen automatischen Dosierung von Beduftungs- und/oder Desodorierungsmitteln ist zudem die Dosierung, insbesondere für einen Nutzer wesentlich erleichtert, da keine Eingabe, zum Beispiel zur Steuerung und/oder Regelung des Dosiergerätes seitens des Benutzers erforderlich ist.

Insbesondere kann es sich bei der Steuereinheit um einen programmierbaren Mikroprozessor handeln. In einer beispielhaften Ausführungsform ist auf dem Mikroprozessor eine Mehrzahl von Dosierprogrammen gespeichert, die ein Ausgeben von entsprechenden in den mindestens zwei Kartuschen aufnehmbaren Beduftungs- und/oder Desodorierungsmitteln auslösen können.

Die Steuereinheit weist in einer beispielhaften Ausführungsform keine Verbindung zur möglicherweise vorhandenen Steuerung des Haushaltsgeräts auf. Es werden demnach keine Informationen, insbesondere elektrische und/oder elektromagnetischen Signale, direkt zwischen der Steuereinheit und der Steuerung des Haushaltsgeräts ausgetauscht.

In einer alternativen Ausgestaltung kann die Steuereinheit mit der vorhandenen Steuerung des Reinigungsgeräts gekoppelt sein. Bevorzugt ist diese Kopplung kabellos ausgeführt. Beispielsweise kann ein Signal drahtlos an das Dosiergerät übertragen werden, wenn die Steuerung der Geschirrspülmaschine die Beduftung- und/oder Desodorierung bewirkt.

In der Steuereinheit können mehrere Programme zur Freigabe von unterschiedlichen Beduftungs- und/oder Desodorierungsmitteln gespeichert sein.

Der Aufruf des entsprechenden Programms kann in einer beispielhaften Ausgestaltung durch entsprechende RFID-Label oder am Behälter ausgeformte geometrische Informationsträger bewirkt sein. So ist es beispielsweise möglich, die gleiche Steuereinheit für eine Mehrzahl von Anwendungen zu verwenden, beispielsweise zum Auslösen einer Beduftung- und/oder Desodorierung.

Zum Auslösen einer Beduftung- und/oder Desodorierung von insbesondere zur Vergelung neigenden Beduftungs- und/oder Desodorierungsmitteln kann die Steuereinheit derart konfiguriert sein, dass einerseits die Beduftung- und/oder Desodorierung in hinreichend kurzer Zeit erfolgt um ein gutes Ergebnis zu gewährleisten und andererseits die Beduftungs- und/oder Desodorierungsmittel nicht so schnell dosiert, dass Vergelungen des Schwalls auftreten. Dies kann beispielsweise durch eine intervallartige Freisetzung realisiert sein, wobei die einzelnen Intervalle so eingestellt sein können, das sich die entsprechend dosierte Menge vollständig während eines Zyklus bzw. einem Auslösen von Beduftung und/oder Desodorierung auflösen kann.

Ein Sensor im Sinne dieser Anmeldung kann eine Einrichtung zur Messung und/oder Ermittlung einer Information, zum Beispiel ein Messgrößenaufnehmer oder Messfühler sein, der bestimmte physikalische oder chemische Eigenschaften und/oder die stoffliche Beschaffenheit seiner Umgebung qualitativ oder als Messgröße quantitativ erfassen kann.

Das Dosiergerät kann in einer beispielhaften Weiterentwicklung eine Einrichtung zur Erfassung von Informationen, zum Beispiel einen Sensor aufweisen, der physikalische, chemische und/oder mechanische Parameter aus der Umgebung des Dosiergeräts bestimmen kann. Die Sensoreinheit kann einen oder mehrere aktive und/oder passive Sensoren zur qualitativen und/oder quantitativen Erfassung mechanischer, elektrischer, physikalischer und/oder chemischer Größen umfassen, die als Informationen an die Steuereinheit geleitet werden.

Ein Geruchssensor kann beispielsweise ein oder mehrere elektrochemische Sensoren umfassen oder aus diesen gebildet sein, die in der Lage sind, die Anwesenheit bestimmter Duftstoffe bzw. Schlechtgerüche zu ermitteln. Insbesondere können dies beispielsweise Sensoren sein, die schwefelhaltige Riechstoffe, flüchtige Carbonsäure, flüchtige Kohlenwasserstoffe und/oder stickstoffhaltige Verbindungen erfassen können. Derartige Sensoren können beispielsweise eine Fläche mit signalgebenden Bindemolekülen aufweisen. Diese signalgebenden Bindemoleküle können über ein chemisches und/oder physikalisches Backbone an einen Signaltransmitter, wie beispielsweise einen Quatenbot, einen Nanopartikel, eine Mizelle, ein Vesikel oder eine Membran gebunden sein.

Eine Datenleitung zwischen einer Einrichtung zur Messung oder Ermittlung von Informationen, zum Beispiel vorstehend beschriebene Einrichtungen, und der Steuereinheit kann über ein elektrisch leitendes Kabel oder kabellos realisiert sein.

Eine kabellos ausgebildete Datenleitung ist insbesondere durch die Übertragung elektromagnetischer Wellen ausgebildet. Es ist bevorzugt, eine kabellose Datenleitung nach normierten Standards wie beispielsweise Bluetooth, IrDA, IEEE 802, Zigbee, NFC etc. auszubilden.

In einer beispielhaften Ausbildung ist die Sensoreinheit am Boden des Dosiergeräts angeordnet wobei in Gebrauchsstellung der Boden des Dosiergeräts in Schwerkraftrichtung nach unten gerichtet ist.

Im Sinne dieser Anmeldung wird als wenigstens eine Energiequelle ein Bauelement des Dosiergerätes verstanden, welches zweckmäßig ist, eine zum Betrieb des Dosiergerätes geeignete Energie bereit zu stellen. Bevorzugt umfasst das Dosiergerät wenigstens eine Energiequelle und ist die wenigstens eine Energiequelle derart ausgestaltet, dass das Dosiergerät autark insbesondere von einer externen Energiequelle ist.

Vorzugsweise stellt die wenigstens eine Energiequelle elektrische Energie zur Verfügung. Bei der Energiequelle kann es sich beispielsweise um eine Batterie, einen Akkumulator, ein Netzgerät, eine Solarzelle oder dergleichen handeln.

In einer beispielhaften Ausgestaltung ist die Energiequelle austauschbar ausgeführt, zum Beispiel in Form einer auswechselbaren Batterie.

Eine Batterie kann beispielsweise ausgewählt sein aus der Gruppe der Alkali-Mangan-Batterien, Zink-Kohle-Batterien, Nickel-Oxyhydroxid-Batterien, Lithium-Batterien, Lithium-Eisensulfid-Batterien, Zink-Luft-Batterien, Zink-Chlorid-Batterien, Quecksilberoxid-Zink-Batterien und/oder Silberoxid-Zink-Batterien.

Als Akkumulator eignen sich beispielsweise Bleiakkumulatoren (Bleidioxid/Blei), Nickel-Cadmium-Akkus, Nickel-Metallhydrid-Akkus, Lithium-Ionen-Akkus, Lithium-Polymer-Akkus, Alkali-Mangan-Akkus, SilberZink-Akkus, Nickel-Wasserstoff-Akkus, Zink-Brom-Akkus, Natrium-Nickelchlorid-Akkus und/oder NickelEisen-Akkus.

Der Akkumulator kann insbesondere in derart ausgestaltet sein, dass er durch Induktion wiederaufladbar ist.

Es ist jedoch auch denkbar, mechanische Energiequellen bestehend aus einer oder mehrerer Schraubenfeder, Torsionsfeder oder Drehstabfeder, Biegefeder, Luftfeder/Gasdruckfeder und/oder Elastomerfeder auszubilden.

Die Energiequelle ist in dergestalt dimensioniert, dass das Dosiergerät in etwa 300 Beduftungs- und/oder Desodorierungszyklen durchlaufen kann, bevor die Energiequelle erschöpft ist. Es ist insbesondere bevorzugt, dass die Energiequelle zwischen 1 und 300 Zyklen, ganz besonders bevorzugt zwischen 10 und 300, weiterhin bevorzugt zwischen 100 und 300 durchlaufen kann, bevor die Energiequelle erschöpft ist.

Ferner können in oder an der Dosiergerät Mittel zur Energieumwandlung vorgesehen sein, die eine Spannung erzeugen, mittels derer der Akkumulator aufgeladen wird. Beispielsweise können diese Mittel als Dynamo ausgebildet sein, der durch die Wasserströme während eines Spülgangs in einer Geschirrspülmaschine angetrieben wird und die so erzeugte Spannung an den Akkumulator abgibt.

In einer weiteren beispielhaften Ausführung weist das Dosiergerät wenigstens einen Schwingzerstäuber auf, über den es ermöglicht ist, ein Beduftungs- und/oder Desodorierungsmittel in die Gasphase zu überführen bzw. in der Gasphase zu halten. So ist es beispielsweise denkbar, Beduftungs- und/oder Desodorierungsmittels mittels des Schwingzerstäubers zu verdampfen, zu vernebeln und/oder zu zerstäuben, wodurch das Beduftungs- und/oder Desodorierungsmittel in die Gasphase übergeht bzw. ein Aerosol in der Gasphase bildet, wobei die Gasphase üblicherweise Luft ist.

Insbesondere von Vorteil ist diese Ausführung bei der Anwendung in einer Geschirrspülmaschine, in welcher eine entsprechende Freisetzung von Beduftungs- und/oder Desodorierungsmitteln in die Gasphase in einem verschließbaren Spül bzw. Waschraum erfolgt. Das in die Gasphase eingebrachte Beduftungs- und/oder Desodorierungsmittel kann sich gleichmäßig im Spülraum verteilen und auf dem in der Geschirrspülmaschine befindlichen Spülgut niederschlagen.

Das durch den Schwingzerstäuber freigesetzte Beduftungs- und/oder Desodorierungsmittel kann ausgewählt sein aus der Gruppe der tensidhaltigen Beduftungs- und/oder Desodorierungsmittel, enzymhaltigen Beduftungs- und/oder Desodorierungsmittel, geruchsneutralisierenden Beduftungs- und/oder Desodorierungsmittel, biozide Beduftungs- und/oder Desodorierungsmittel, antibakteriellen Beduftungs- und/oder Desodorierungsmittel.

Durch das Aufbringen der Beduftungs- und/oder Desodorierungsmittel auf das Spülgut aus der Gasphase wird eine gleichmäßige Schicht der entsprechenden Beduftungs- und/oder Desodorierungsmittel auf der Spülgutoberfläche aufgebracht. Besonders bevorzugt ist es, dass die gesamte Spülgutoberfläche von dem Beduftungs- und/oder Desodorierungsmittel benetzt ist.

Hierdurch kann z.B. eine Wirkung vordem Beginn eines Wasser freisetzenden Reinigungsprogramms einer Geschirrspülmaschine erzielt werden. Beispielsweise kann durch ein geeignetes Beduftungs- und/oder Desodorierungsmittel ein Entstehen von Schlechtgerüchen durch biologische Zersetzungsprozesse von an dem Spülgut anhaftenden Speiseresten unterdrückt werden.

Ferner ist es möglich nach der Beendigung eines Reinigungsprogramms einer Geschirrspülmaschine ein Beduftungs- und/oder Desodorierungsmittel mittels des Schwingzerstäubers auf das Spülgut aufzubringen. Hierbei kann es sich beispielsweise um ein antibakteriell wirkendes Beduftungs- und/oder Desodorierungsmittel oder ein Beduftungs- und/oder Desodorierungsmittel zur Modifikation von Oberflächen handeln.

Die gegenständliche Aufgabe wird insbesondere durch die Verwendung eines Dosiergerätes im Inneren einer Geschirrspülmaschine gelöst.

In einer weiteren Ausgestaltung ist mindestens eine der Vorrichtungen zur Durchführung eines der im nachfolgenden beschriebenen Verfahren, die mit dem Dosiergerät ausführbar und/oder steuerbar sind, ein mobiles Gerät. Insbesondere kann eine Kommunikation über ein Kommunikationssystem zwischen einem mobilen Gerät, beispielsweise einem Smartphone, Laptop, Laptop, Tablet, Wearable, Computational Engine und mindestens einer weiteren Vorrichtung, beispielsweise einem Server vorgenommen werden.

### Gemäß einer beispielhaften Ausgestaltung umfasst das Dosiergerät eine

Kommunikationsschnittstelle. Beispielsweise ist die Kommunikationsschnittstelle für eine drahtgebundene oder drahtlose Kommunikation eingerichtet. Beispielsweise ist die Kommunikationsschnittstelle eine Netzwerkschnittstelle. Die Kommunikationsschnittstelle ist beispielsweise dazu eingerichtet mit einem Kommunikationssystem zu kommunizieren. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetz, ein Telefonnetzwerk und/oder das Internet. Ein Kommunikationssystem kann die Kommunikation mit einem externen Computer umfassen, beispielsweise über eine Internetverbindung.

Gemäß einem beispielhaften Aspekt umfasst das Dosiergerät zumindest einen Prozessor und zumindest einen Speicher mit Computerprogrammcode, wobei der zumindest eine Speicher und der Computerprogrammcode dazu eingerichtet sind, mit dem zumindest einen Prozessor zumindest ein Verfahren nach den im nachfolgenden beschriebenen Aspekten auszuführen und/oder zu steuern. Unter einem Prozessor soll zum Beispiel eine Kontrolleinheit, ein Mikroprozessor, eine Mikrokontrolleinheit wie ein Mikrocontroller, ein digitaler Signalprozessor (DSP), eine anwendungsspezifische integrierte Schaltung (ASIC) oder ein Field Programable Gate Array (FPGA) verstanden werden.

Zum Beispiel umfasst ein beispielhaftes Dosiergerät ferner Mittel zum Speichern von Informationen wie einen Programmspeicher und/oder einen Hauptspeicher. Zum Beispiel umfasst ein beispielhaftes Dosiergerät ferner jeweils Mittel zum Empfangen und/oder Senden von Informationen über ein Netzwerk wie eine Netzwerkschnittstelle.

Ein beispielhaftes Dosiergerät ist oder umfasst etwa eine Datenverarbeitungsanlage, die softwaremäßig und/oder hardwaremäßig eingerichtet ist, um die jeweiligen Schritte eines beispielhaften Verfahrens gemäß Aspekten ausführen zu können. Beispiele für eine Datenverarbeitungsanlage sind ein Computer, ein Desktop-Computer, ein Server, ein Thinclient, eine Computational Engine und/oder ein tragbarer Computer (Mobilgerät), wie etwa ein Laptop-Computer, ein Tablet-Computer, ein Wearable, ein persönlicher digitaler Assistent oder ein Smartphone.

Die zuvor in dieser Beschreibung beschriebenen beispielhaften Ausgestaltungen sollen auch in allen Kombinationen miteinander offenbart verstanden werden. Insbesondere sollen beispielhafte Ausgestaltungen in Bezug auf die unterschiedlichen Aspekten offenbart verstanden werden.

Insbesondere sollen durch die vorherige oder folgende Beschreibung von Verfahrensschritten gemäß bevorzugter Ausführungsformen eines Verfahrens auch entsprechende Mittel zur Durchführung der Verfahrensschritte durch bevorzugte Ausführungsformen einer Vorrichtung offenbart sein. Ebenfalls soll durch die Offenbarung von Mitteln einer Vorrichtung zur Durchführung eines Verfahrensschrittes auch der entsprechende Verfahrensschritt offenbart sein.

Weitere vorteilhafte beispielhafte Ausgestaltungen sind der folgenden detaillierten Beschreibung einiger beispielhafter Ausführungsformen, insbesondere in Verbindung mit den Figuren, zu entnehmen. Die Figuren sollen jedoch nur dem Zwecke der Verdeutlichung, nicht aber zur Bestimmung des Schutzbereiches dienen. Die Figuren sind nicht maßstabsgetreu und sollen lediglich das allgemeine Konzept beispielhaft widerspiegeln. Insbesondere sollen Merkmale, die in den Figuren enthalten sind, keineswegs als notwendiger Bestandteil erachtet werden.

### Kurze Beschreibung der Figuren

In der Zeichnung zeigt
- Fig. 1: eine schematische Darstellung einer beispielhaften Ausführungsform eines Systems gemäß dem fünften Aspekt;
- Fig. 2:: ein Flussdiagramm einer beispielhaften Ausgestaltung eines Verfahrens gemäß dem ersten Aspekt;
- Fig. 3:: ein Flussdiagramm einer beispielhaften Ausgestaltung eines Verfahrens gemäß dem ersten Aspekt;
- Fig. 4: ein Flussdiagram eines Teilaspekt einer beispielhaften Ausgestaltung eines Verfahrens gemäß dem ersten Aspekt;
- Fig. 5:: eine schematische Darstellung einer beispielhaften Ausführungsform einer Vorrichtung gemäß dem zweiten, dritten oder vierten Aspekt;
- Fig. 6: eine schematische Darstellung eines Aufbaus von einem Geruchssensor; und
- Fig. 7a-f: beispielhafte Diagramme von einem Geruchssensor erfassten Informationen.

### Detaillierte Beschreibung einiger beispielhafter Ausführungsformen

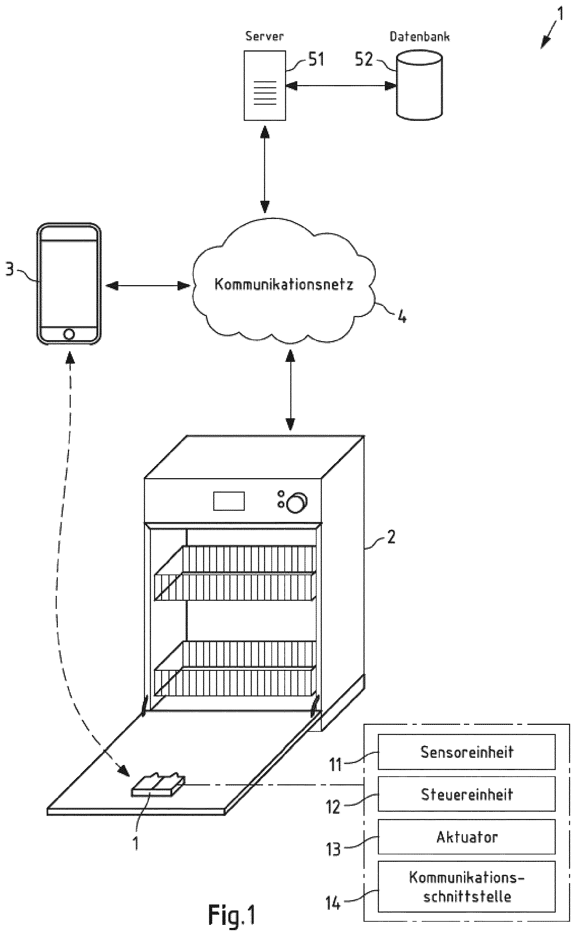

Fig. 1 ist eine schematische Darstellung einer beispielhaften Ausführungsform eines Systems 1. Das System umfasst ein Dosiergerät 1, ein Reinigungsgerät 2 (z.B. eine Geschirrspülmaschine), ein mobiles Gerät 3 (z.B. ein Smartphone), ein Kommunikationsnetz 4 und eine (zentrale oder dezentrale) Rechen- und Speichereinrichtung. Die Rechen- und Speichereinrichtung umfasst vorliegend einen Server 51 und eine Datenbank 52.

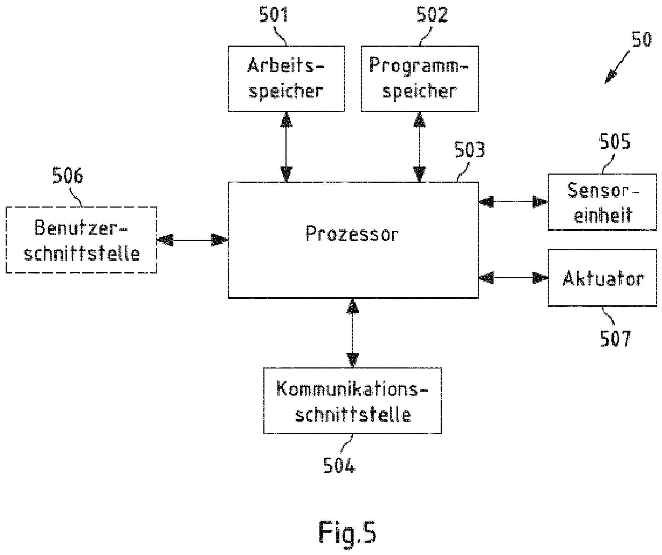

Das Dosiergerät 1, das Reinigungsgerät 2, das Smartphone 3, die Rechen- und Speichereinrichtung können jeweils beispielsweise durch die Vorrichtung 50 nach Fig. 5 repräsentiert sein.

Vorliegend ist das Dosiergerät 1 an einer Schließeinrichtung des Reinigungsgerätes 2 angeordnet. Alternativ kann das Dosiergerät beweglich ausgebildet sein, so dass das Dosiergerät 1 beispielsweise frei in dem Innenraum des Reinigungsgerätes 2 positionierbar ist. Das Dosiergerät 1 muss beispielsweise derart zu einem Reinigungsgerät 2 positionierbar sein, dass das Dosiergerät 1 zum einen eine Geruchsbelastung in dem Innenraum des Reinigungsgerätes 2 erfassen kann, und zum anderen eine Beduftung und/oder Desodorierung derart auslösen kann, dass freigesetztes Beduftungs- und/oder Desodorierungsmittel in dem Innenraum des Reinigungsgerätes 2 verteilbar ist. Ansonsten kann beispielsweise keine Beseitigung einer Geruchsbelastung im Innenraum des Reinigungsgerätes 2 durch das Dosiergerät 1 erfolgen.

Das Dosiergerät 1 umfasst eine Sensoreinheit 11, eine Steuereinheit 12, einen Aktuator 13 und eine Kommunikationsschnittstelle 14. Ferner umfasst das Dosiergerät 1 z.B. eine mit dem Dosiergerät 1 koppelbare Kartusche zur Bevorratung von mindestens einer Zubereitung eines Beduftungs- und/oder Desodorierungsmittels.

Die Sensoreinheit 11 umfasst mindestens einen Geruchssensor, wobei ein Erfassen von ersten Informationen indikativ für eine Geruchsbelastung im Innenraum des Reinigungsgerätes 2 mit diesem möglich ist.

Mittels der Steuereinheit 12 ist eine Beduftungs- und/oder Desodorierungsinformation erzeugbar, wobei die Beduftungs- und/oder Desodorierungsinformation basierend auf von der Sensoreinheit 11 erfassten ersten Informationen erzeugbar ist.

Der Aktuator 13 ist derart eingerichet, dass eine Beduftung und/oder Desodorierung auslösbar ist. Die Beduftung und/oder Desodorierung erfolgt entsprechend der von der Steuereinheit 12 erzeugten Beduftungs- und/oder Desodorierungsinformation. Mittels des Aktuators 13 ist entsprechend der Beduftungs- und/oder Desodorierungsinformation mindestens eine Zubereitung eines Beduftungs- und/oder Desodorierungsmittels (z.B. das Beduftungs- und/oder Desodorierungsmittel, welches in der Kartusche bevorratet ist) freisetzbar. Mittels der Freisetzung des Beduftungs- und/oder Desodorierungsmittels ist eine Geruchsbelastung im Innenraum des Reinigungsgerätes 2 beseitigbar.

Die Rechen- und Speichereinrichtung umfasst einen Server 51 und eine Datenbank 52. Der Server 51 und die Datenbank 52 sind vorliegend separiert voneinander und über eine Kommunikationsverbindung zum Austausch von Daten miteinander verbunden. Der Server 51 und die Datenbank 52 sind in einer alternativen Ausgestaltung in einer Einrichtung zusammen eingerichtet.

Das Dosiergerät 1, das Smartphone 3, die Rechen- und Speichereinrichtung und das Reinigungsgerät 2 sind über das Kommunikationsnetz 4 miteinander verbunden. Entsprechend können das Dosiergerät 1, das Smartphone 3, die Rechen- und Speichereinrichtung, und das Reinigungsgerät 2 entweder direkt untereinander (wie z.B. durch den gestrichelten Pfeil zwischen dem Dosiergerät 1 und dem Smartphone 3 angedeutet) beispielsweise eine Kommunikationsverbindung aufbauen. Zusätzlich oder alternativ können das Dosiergerät 1, das Smartphone 3, die zentrale Rechen- und Speichereinrichtung, und das Reinigungsgerät 2 jeweils untereinander Kommunikationsverbindungen zum Austausch von Daten über das Kommunikationsnetz 4 aufbauen und entsprechend nutzen. Als Kommunikationsverbindungen können beispielsweise Nahbereichsfunkverbindungen (z.B. mittels Bluetooth) oder drahtlose Funkverbindungen (z.B. mittels WLAN, SubGHz mit einem privaten oder öffentlichen Netzwerk) verwendet werden. Beispielsweise kann das Dosiergerät 1 mit einem öffentlichen oder privaten Netz, wie z.B. Kommunikationsnetz 4, und/oder einem Endgerät, z.B. einem PC, Smartphone (z.B. Smartphone 3) oder einem interaktiven, sozialen Kommunikationssystem (z.B. Amazon Echo) eine Kommunikationsverbindung aufbauen und entsprechend nutzen. Exemplarisch umfasst das Dosiergerät 1 vorliegend zur Nutzung von einer der vorgenannten Kommunikationsverbindungen eine Kommunikationsschnittstelle 14. Die weiteren Entitäten (z.B. das Reinigungsgerät 2, das Smartphone 3, und/oder die Rechen- und Speichereinrichtung) umfassen beispielsweise eine entsprechende Kommunikationsschnittstelle.

Das Dosiergerät 1 kann beispielsweise in das Reinigungsgerät 2 integriert sein. Alternativ ist das Dosiergerät 1 ein mobiles Gerät. Als mobiles Gerät ausgebildet ist das Dosiergerät im Wesentlichen frei in dem Innenraum des Reinigungsgerätes positionierbar. Das Dosiergerät 1 kann autark arbeiten, also beispielsweise ohne Anbindung an das Kommunikationsnetz 4 ausgebildet sein. Eine Rückmeldung über einen Betriebszustand des Dosiergerätes 1 und/oder zusätzlich über das Reinigungsgerät 2, kann beispielsweise direkt am Dosiergerät 1 erfolgen, z.B. mittels einer Anzeigevorrichtung (z.B. Wiedergabe von zumindest einem Lichtsignal, akustischen Signal und/oder optischen Signal). Das Dosiergerät kann optional eine derartige Anzeigevorrichtung (z.B. ein Display) aufweisen oder umfassen.

Das System 1 ermöglicht eine Beduftung, eine Desodorierung oder eine kombinierte Beduftung und Desodorierung eines Innenraums eines Reinigungsgerätes, insbesondere zur Beseitigung einer in dem Innenraum des Reinigungsgerätes herrschenden Geruchsbelastung, z.B. des Innenraums des Reinigungsgerätes 2.

In einer beispielhaften Ausgestaltung werden von der Sensoreinheit 11 des Dosiergerätes 1 erste Informationen erfasst. Diese erfassten ersten Informationen werden über das Kommunikationsnetz 4 an den Server 51 der Rechen- und Speichereinrichtung übermittelt. Der Server erzeugt basierend auf den erfassten ersten Informationen eine Beduftungs- und/oder Desodorierungsinformation und übermittelt diese über das Kommunikationsnetz 4 an das Dosiergerät 1. Basierend auf der erzeugten Beduftungs- und/oder Desodorierungsinformation erfolgt seitens des Dosiergerätes 1 das Auslösen einer Beduftung und/oder Desodorierung des Innenraums des Reinigungsgerätes 1. Das Dosiergerät 1 kann beispielsweise eine Kommunikationsverbindung zu dem Smartphone 3 aufbauen, beispielsweise nach einer erfolgten Anfrage zum Aufbau der Kommunikationsverbindung, die das Dosiergerät 1 von dem Smartphone 3 erhalten hat. Über die aufgebaute Kommunikationsverbindung können einem Nutzer des Smartphones 3 insbesondere Informationen über den olfaktorischen Zustand des Innenraums des Reinigungsgerätes 2 übermittelt werden, z.B. ob eine mitunter als unangenehm durch den Nutzer empfundene Geruchsbelastung im Innenraum des Reinigungsgerätes 2 vorliegt oder nicht. Beispielsweise kann eine einfache Beschreibung wie "Schlechtgeruch wurde erkannt" an das Smartphone 3 übermittelt werden. Auch komplexere Zustandsinformationen, wie beispielsweise "Im Geschirrspüler riecht es nach saurer Milch oder Essig" können an das Smartphone 3 übermittelt werden. Im Anschluss kann der Nutzer beispielsweise ein Auslösen einer Beduftung und/oder Desodorierung des Innenraums des Reinigungsgerätes 2 veranlassen. Hierzu kann beispielsweise eine Beduftungs- und/oder Desodorierungsinformation, die in diesem Fall von dem Smartphone 3 erzeugt wird, an das Dosiergerät 1 übermittelt werden. Alternativ oder zusätzlich kann das Dosiergerät 1 (insbesondere) automatisch eine Beduftung und/oder Desodorierung auslösen.

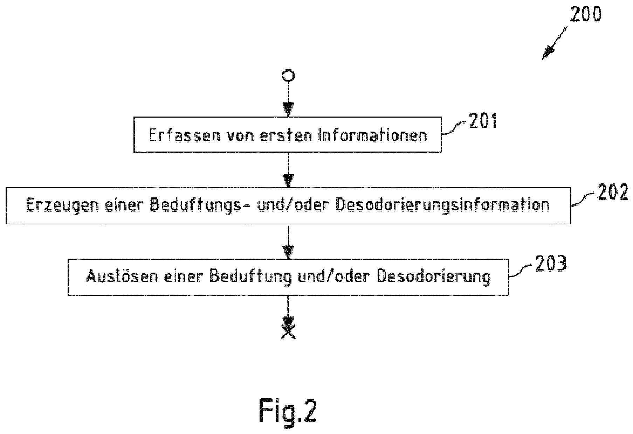

Fig. 2 stellt ein Flussdiagramm 200 einer beispielhaften Ausführungsform eines Verfahrens dar, das im Kontext nach den beschriebenen Aspekten ausgeführt werden kann. Das Verfahren wird beispielsweise von der Steuereinheit 12 des Dosiergerätes 1 der Fig. 1 ausgeführt, die beispielsweise als Vorrichtung 50 der Fig. 5 ausgebildet sein kann.

In einem ersten Schritt 201 werden erste Informationen erfasst. Die ersten Informationen sind indikativ für eine Geruchsbelastung im Innenraum eines Reinigungsgerätes, wobei die ersten Informationen mit mindestens einem Geruchssensor erfasst werden. Der Geruchssensor ist beispielsweise von der Sensoreinheit 11 des Dosiergerätes 1 nach Fig. 1 umfasst.

In einem zweiten Schritt 202 wird eine Beduftungs- und/oder Desodorierungsinformation erzeugt. Das Erzeugen basiert zumindest teilweise auf den im Schritt 201 erfassten ersten Informationen.

In einem dritten Schritt 203 erfolgt ein Auslösen einer Beduftung und/oder Desodorierung. Das Auslösen der Beduftung und/oder Desodorierung erfolgt mittels eines Aktuators (z.B. Aktuator 13 des Dosiergerätes 1 nach Fig. 1). Der Aktuator ist eingerichtet, basierend auf der Beduftungs- und/oder Desodorierungsinformation Beduftungs- und/oder Desodorierungsmittel freizusetzen.

Das Flussdiagramm 200 kann beispielsweise in regelmäßigen Zeitintervallen ausgeführt werden. So kann sichergestellt werden, dass eine Behandlung bzw. Maßnahme zur Beseitigung von einer vorhandenen Geruchsbelastung durchgeführt wird. Durch ein mehrmaliges Ausführen des Flussdiagramms 200 kann folglich eine angepasste Beseitigung der Geruchsbelastung entweder durch eine Beduftung, oder durch eine Desodorierung oder durch eine Kombination von einer Beduftung und Desodorierung durchgeführt werden.

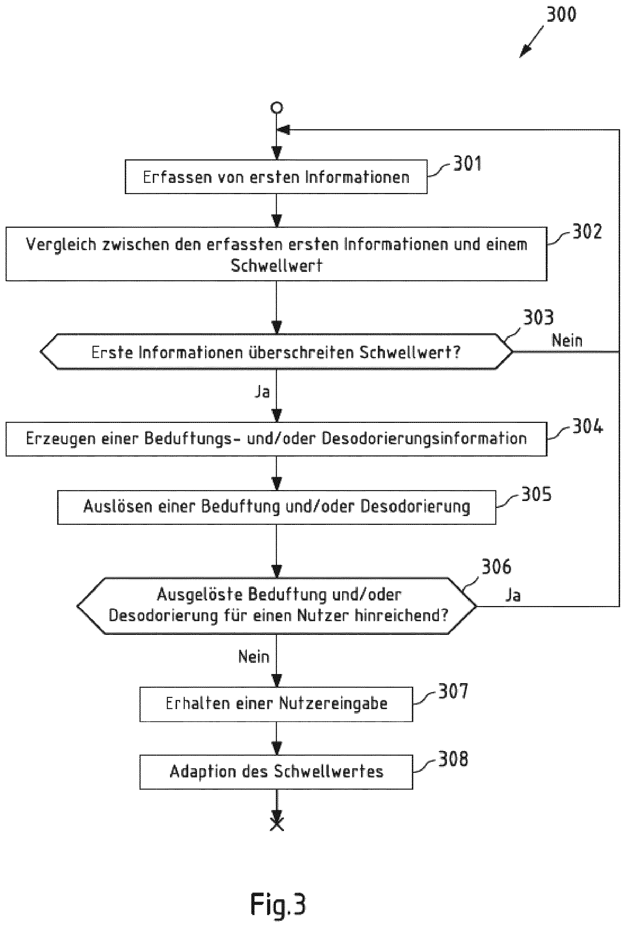

Fig. 3 stellt ein Flussdiagramm 300 einer beispielhaften Ausführungsform eines Verfahrens dar, das im Kontext der beschriebenen Aspekte ausgeführt werden kann. Das Verfahren wird beispielsweise von der Steuereinheit 12 des Dosiergerätes 1 der Fig. 1 ausgeführt, die beispielsweise als Vorrichtung 50 der Fig. 5 ausgebildet sein kann.

In einem ersten Schritt 301 erfolgt ein Erfassen von ersten Informationen. Die ersten Informationen sind indikativ für eine Geruchsbelastung im Innenraum eines Reinigungsgerätes, wobei die ersten Informationen mit mindestens einem Geruchssensor erfasst werden. Der Geruchssensor ist beispielsweise von der Sensoreinheit 11 des Dosiergerätes 1 nach Fig. 1 umfasst.

In einem zweiten Schritt 302 erfolgt ein Vergleich zwischen den erfassten ersten Informationen und einem vordefinierten Schwellwert. Die erfassten ersten Informationen und/oder der vordefinierte Schwellwert sind indikativ für zumindest einen der folgenden Parameter:
- Intensität der Geruchsbelastung im Innenraum des Reinigungsgerätes;
- Art der Geruchsbelastung im Innenraum des Reinigungsgerätes;
- Dynamik der Geruchsbelastung im Innenraum des Reinigungsgerätes;
- oder eine Kombination hiervon.

In einem dritten Schritt 303 wird überprüft, ob die erfassten ersten Informationen den Schwellwert überschreiten. Für den Fall, dass der Schwellwert von den erfassten ersten Informationen nicht überschritten wird, ist die im Innenraum des Reinigungsgerätes herrschende Geruchsbelastung noch nicht derart ausgeprägt, dass diese von einem Nutzer als störend bzw. unangenehm empfunden wird. Dieser Fall kann beispielsweise vorliegen, wenn die Geruchsbelastung noch sehr gering ist oder wenn das Reinigungsgerät sich gerade im Leer- und Sauberzustand befindet. Für den weiteren Fall, dass die erfassten ersten Informationen den vordefinierten Schwellwert überschreiten, wird im Schritt 304 eine Beduftungs- und/oder Desodorierungsinformation erzeugt. Die Beduftungs- und/oder Desodorierungsinformation ist in diesem Fall indikativ für eine Geruchsbelastung im Innenraum des Reinigungsgerätes, die von einem Nutzer mitunter als störend bzw. unangenehm empfunden werden kann.

In einer alternativen Ausgestaltung des Flussdiagramms 300 kann Schritt 304, wonach ein Erzeugen einer Beduftungs- und/oder Desodorierungsinformation erfolgt, vor dem Schritt 303 ausgeführt werden, so dass stets - unabhängig vom Vergleichsergebnis des Schrittes 302 - eine Beduftungs- und/oder Desodorierungsinformation erzeugt wird. Die erzeugte Beduftungs- und/oder Desodorierungsinformation ist beispielsweise indikativ für eine von einem Nutzer als unangenehm empfundene Geruchsbelastung im Innenraum des Reinigungsgerätes oder indikativ für eine von einem Nutzer als nicht unangenehm empfundene Geruchsbelastung im Innenraum des Reinigungsgerätes. Anschließend kann überprüft werden, ob die erfassten ersten Informationen den vordefinierten Schwellwert überschreiten, um entscheiden zu können, ob ein Auslösen einer Beduftung und/oder Desodorierung zur Beseitigung einer mitunter vorhandenen unangenehmen Geruchsbelastung im Innenraum des Reinigungsgerätes durchgeführt werden soll oder nicht.

Basierend auf der in Schritt 304 erzeugten Beduftungs- und/oder Desodorierungsinformation erfolgt in Schritt 305 das Auslösen einer Beduftung und/oder Desodorierung. Beispielsweise erfolgt das Auslösen der Beduftung und/oder Desodorierung, wenn die erzeugte Beduftungs- und/oder Desodorierungsinformation indikativ für eine von einem Nutzer als unangenehm empfundene Geruchsbelastung im Innenraum des Reinigungsgerätes ist. Das Auslösen der Beduftung und/oder Desodorierung kann beispielsweise unterschiedliche Beduftungs- und/oder Desodorierungsmittel in Abhängigkeit der Art der Geruchsbelastung im Innenraum des Reinigungsgerätes freisetzen. Alternativ oder zusätzlich kann das Auslösen der Beduftung und/oder Desodorierung unterschiedliche Mengen eines Beduftungs- und/oder Desodorierungsmittels freisetzen, z.B. in Abhängigkeit der Intensität der Geruchsbelastung im Innenraum des Reinigungsgerätes.

In Schritt 306 erfolgt eine Überprüfung, ob die ausgelöste Beduftung und/oder Desodorierung für einen Nutzer hinreichend war oder nicht, z.B. ob eine von dem Nutzer als unangenehm empfundene Geruchsbelastung im Innenraum des Reinigungsgerätes durch die ausgelöste Beduftung und/oder Desodorierung beseitigt wurde oder nicht. Ziel dieser Überprüfung kann es beispielsweise sein, den Nutzer zu befragen, ob die Behandlung der Geruchsbelastung im Innenraum des Reinigungsgerätes durch die ausgelöste Beduftung und/oder Desodorierung ausreichend war (z.B. die Intensität der Geruchsbelastung im Innenraum des Reinigungsgerätes ausreichend reduziert wurde), ob sie angenehm war (z.B. im Sinne der Hedonik, ob der Eigengeruch des freigesetzten Beduftungs- und/oder Desodorierungsmittels für den Nutzer angenehm war), und/oder ob die ausgelöste Beduftung und/oder Desodorierung ggf. nochmals wiederholt werden soll (z.B. mit veränderter Intensität und/oder Menge an freigesetztem Beduftungs- und/oder Reinigungsmittel). War die ausgelöste Beduftung und/oder Desodorierung für den Nutzer hinreichend, kann das Flussdiagramm 300 erneut durchlaufen werden. Andernfalls kann beispielsweise mit dem Schritt 307 fortgefahren werden.

In Schritt 307 wird eine Nutzereingabe erhalten. Die Nutzereingabe kann beispielsweise auf Basis einer an den Nutzer übermittelten Frage erfolgen. Die Nutzereingabe kann beispielsweise repräsentieren, ob die Behandlung der Geruchsbelastung im Innenraum des Reinigungsgerätes durch die ausgelöste Beduftung und/oder Desodorierung ausreichend war, ob sie angenehm war, ob die ausgelöste Beduftung und/oder Desodorierung ggf. wiederholt werden soll, oder eine Kombination hiervon. Alternativ kann die Nutzereingabe in dem Schritt 307 erfasst werden.

Das Erfassen der Nutzereingabe umfasst beispielsweise das maschinelle Erfassten einer Information, beispielsweise eine Eingabe auf einer Eingabevorrichtung, die seitens des Nutzers durchgeführt wird, z.B. mittels einer Tasteneingabe auf einer Tastatur, und/oder auf einer berührungsempfindlichen Anzeigevorrichtung. Das Erfassen der Nutzereingabe kann beispielsweise mit Mitteln zur Erfassung der Nutzereingabe stattfinden, die beispielsweise Teil der Vorrichtung (z.B. Vorrichtung 50 nach Fig. 5) oder des Systems (z.B. System 1 nach Fig. 1) sind. Wird die Nutzereingabe erhalten, wie z.B. in dem Schritt 307, ist die erhaltene Nutzereingabe beispielsweise wie zuvor beschrieben erfasst worden.

In Schritt 308 erfolgt ein Adaptieren des vordefinierten Schwellwertes. Das Adaptieren des vordefinierten Schwellwertes erfolgt beispielsweise basierend auf der erhaltenen oder erfassten Nutzereingabe. Der zum Überprüfen herangezogene vordefinierte Schwellwert, kann entsprechend der erhaltenen oder erfassten Nutzereingabe adaptiert (z.B. variiert) werden. Durch das Adaptieren des vordefinierten Schwellwertes wird beispielsweise beim nächsten Durchlaufen des Flussdiagramms 300 der in dem Schritt 302 durchgeführte Vergleich zu einem anderen Ergebnis führen, als wenn der Vergleich in dem Schritt 302 mit einem vordefinierten Schwellwert durchgeführt werden würde, der nicht entsprechend der erhaltenen oder erfassten Nutzereingabe (Schritt 307) adaptiert wurde. Ist die erhaltene oder erfasste Nutzereingabe beispielsweise indikativ dafür, dass trotz ausgelöster Beduftung und/oder Desodorierung die Beseitigung der Geruchsbelastung im Innenraum des Reinigungsgerätes als nicht ausreichend empfunden wurde, kann das Adaptieren des vordefinierten Schwellwertes diesen Schwellwert herabsetzen. Entsprechend wird beispielsweise eine erhöhte Menge an Beduftungs- und/oder Desodorierungmittel im Rahmen des Auslösens einer Beduftung und/oder Desodorierung freigesetzt oder vice versa.

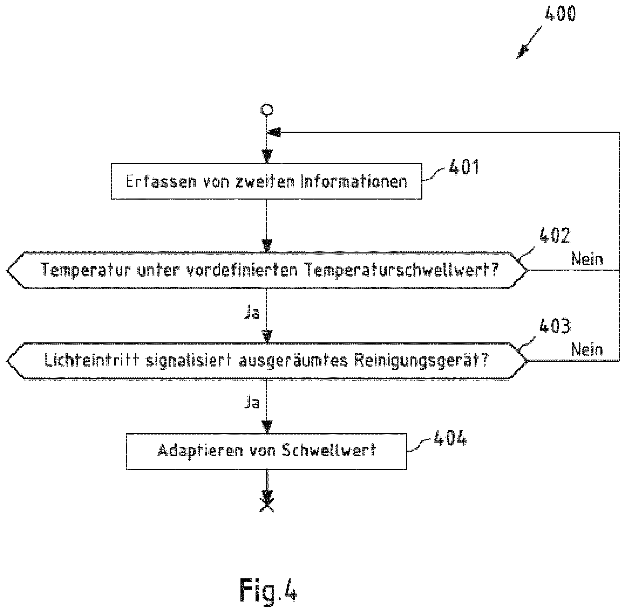

Fig. 4 stellt ein Flussdiagramm 400 einer beispielhaften Ausführungsform eines Verfahrens dar, das im Kontext der beschriebenen Aspekte ausgeführt werden kann. Das Verfahren wird beispielsweise von der Steuereinheit 12 des Dosiergerätes 1 der Fig. 1 ausgeführt, die beispielsweise als Vorrichtung 50 der Fig. 5 ausgebildet sein kann.

Das Flussdiagramm 400 wird beispielsweise im Kontext der "Kalibrierung" von der Steuereinheit 12 des Dosiergerätes 1 nach Fig. 1 durchgeführt. Damit das Erfassen von ersten Informationen indikativ für eine Geruchsbelastung im Innenraum des Reinigungsgerätes zuverlässig funktioniert, muss beispielsweise eine regelmäßige Kalibrierung erfolgen. Bei der Kalibrierung wird beispielsweise der zum Vergleich mit den erfassten ersten Informationen herangezogene vordefinierte Schwellwert festgelegt. Im Folgenden ist exemplarisch eine sogenannte Nullpunkt-Kalibrierung aufgeführt. Die Nullpunkt-Kalibrierung kann beispielsweise im Leer- oder Sauberzustand des Reinigungsgerätes durchgeführt werden und kann insbesondere (automatisch) bei Erkennung des Leer- oder Sauberzustand des Reinigungsgerätes gestartet bzw. getriggert werden, z.B. durch ein entsprechendes Steuersignal des Prozessors.

In Schritt 401 erfolgt ein Erfassen von zweiten Informationen. Die zweiten Informationen sind vorliegend indikativ für einen Lichteintritt und eine Temperatur im Innenraum des Reinigungsgerätes, wobei die zweiten Informationen von mindestens einem Lichtsensor und mindestens einem Temperatursensor erfasst werden.

Um zu erkennen, ob das Reinigungsgerät in einem Leer- oder Sauberzustand ist, wird in Schritt 402 überprüft, ob die Temperatur im Innenraum des Reinigungsgerätes unter einem vordefinierten Temperaturschwellwert liegt. Da bei der Durchführung eines Reinigungszyklus regelmäßig eine erhöhte Temperatur im Innenraum des Reinigungsgerätes herrscht, liegt also für den Fall, dass die Temperatur gemäß den erfassten zweiten Informationen über dem vordefinierten Temperaturschwellwert liegt kein Leer- oder Sauberzustand des Reinigungsgerätes vor. Entsprechend kann das Flussdiagramm 400 erneut durchlaufen werden, so dass beispielsweise für einen späteren Zeitpunkt überprüfbar ist, ob die Temperatur im Innenraum des Reinigungsgerätes unter dem vordefinierten Temperaturschwellwert liegt.

Für den Fall, dass die Temperatur unter dem vordefinierten Schwellwert liegt, wird in Schritt 404 überprüft, ob ein Lichteintritt von dem mindestens einen Lichtsensor erfasst wurde. Über den Lichteintritt in Kombination eines Temperaturabfalls, der über den mindestens einen Temperatursensor erfassbar ist, kann der Lichteintritt signalisieren, dass ein Ausräumen (z.B. von Geschirr) des Reinigungsgerätes stattgefunden hat. Für den Fall, dass der von dem mindestens einen Lichtsensor erfasste Lichteintritt kein ausgeräumtes Reinigungsgerät signalisiert, liegt entsprechend liegt kein Leer- oder Sauberzustand des Reinigungsgerätes vor. Entsprechend kann das Flussdiagramm 400 erneut durchlaufen werden, so dass dies beispielsweise zu einem späteren Zeitpunkt überprüfbar ist.

Wenn die Temperatur im Innenraum des Reinigungsgerätes unter einen bestimmten Temperaturschwellwert gefallen ist, und das Reinigungsgerät ausgeräumt wurde, erfolgt in Schritt 404 ein Adaptieren des Schwellwertes (nicht des Temperaturschwellwertes), so dass eine Nullpunkt-Kalibrierung durchgeführt wird. Hierbei werden die von dem mindestens einen Geruchssensor erfassten ersten Informationen (z.B. absolute von dem mindestens einen Geruchssensor erfasste Messwerte) gespeichert. Diese gespeicherten ersten Informationen dienen beispielsweise der Alterungsüberwachung des mindestens einen Geruchssensors. Die Auswertung dieser gespeicherten ersten Informationen kann beispielsweise dazu genutzt werden, um einen Nutzer des Reinigungsgerätes zu informieren, wann die Empfindlichkeit des mindestens einen Geruchssensors abnimmt und ein eventueller Austausch von diesem erforderlich ist, um eine ordnungsgemäße Funktionsweise der (insbesondere automatischen) Beduftung und/oder Desodorierung sicherstellen zu können.

Die von dem mindestens einen Geruchssensor erfassten ersten Informationen (z.B. absolute von dem mindestens einen Geruchssensor erfasste Messwerte), die im Leer- oder Sauberzustand des Reinigungsgerätes gespeichert werden, charakterisieren einen Zustand des Innenraums des Reinigungsgerätes, der annähernd frei von Störgerüchen ist, also einer neutralen Geruchsbelastung im Innenraum des Reinigungsgerätes. Um zu überprüfen, ob eine neutrale Geruchsbelastung im Innenraum des Reinigungsgerätes vorliegt, kann beispielsweise das Auslösen der Freisetzung von einer definierten Menge von Beduftungs- und/oder Desodorierungsmittel durchgeführt werden. Hierzu muss sichergestellt sein, dass zumindest eins der freigesetzten Beduftungs- und/oder Desodorierungsmittel zu einer Signalvollauslenkung (100%) des mindestens einen Geruchssensors führt, jedoch nicht zu einer Übersättigung von diesem. Für den Fall, dass der mindestens eine Geruchssensor als Ethanolsensor ausgebildet ist, kann beispielsweise als Beduftungs- und/oder Desodorierungsmittel Ethanol oder ein vergleichbar reagierender Alkohol freigesetzt werden. Ist dies nicht möglich, kann alternativ beispielsweise eine Kalibriersubstanz oder eine entsprechende Mischung, die separat bevorratet wird (z.B. im Dosiergerät), verwendet werden oder alternativ können diese von außen zugeführt werden.

Sollte keine Signalvollauslenkung (100%) von dem mindestens einen Geruchssensors erfasst werden, kann eine entsprechende Adaption des Schwellwertes vorgenommen werden, so dass trotz beispielsweise durch Alterung beeinträchtigte Funktion des mindestens einen Geruchssensors weiterhin die Erfassung einer Geruchsbelastung im Innenraum des Reinigungsgerätes mittels des mindestens einen Geruchssensors erfassbar ist.

Fig. 5 zeigt eine schematische Darstellung einer beispielhaften Ausführungsform einer Vorrichtung 50, die im Kontext aller Aspekte eingesetzt werden kann.

Die Vorrichtung 50 kann beispielsweise die Sensoreinheit 11 des Dosiergerätes 1, oder die Steuereinheit 12 des Dosiergerätes 1 der Fig. 1 repräsentieren. Die Vorrichtung 50 kann beispielsweise das Dosiergerät 1 der Fig. 1 repräsentieren.

Die Vorrichtung 50 kann beispielsweise das Flussdiagramm 200 der Fig. 2, Flussdiagramm 300 der Fig. 3 oder Flussdiagramm 400 der Fig. 4 ausführen.

Die Vorrichtung 50 umfasst einen Prozessor 503 mit zugeordnetem Arbeitsspeicher 501 und Programspeicher 502. Der Prozessor 503 führt beispielsweise Programmanweisungen aus, die im Programmspeicher 502 gespeichert sind. Die Programmanweisungen führen das Verfahren gemäß dem ersten Aspekt aus und/oder steuern dieses. Damit enthält der Programmspeicher 502 ein Computerprogramm nach dem fünften Aspekt und stellt ein Computerprogrammprodukt zu dessen Speicherung dar. Vorrichtung 50 stellt ein Beispiel einer Vorrichtung gemäß dem zweiten Aspekt oder eine Vorrichtung eines Systems nach dem dritten Aspekt dar.

Der Programmspeicher 502 kann beispielsweise ein persistenter Speicher, wie beispielsweise ein Read-Only-Memory (ROM)-Speicher sein. Der Programmspeicher 502 kann beispielsweise fest mit dem Prozessor 503 verbunden sein, kann aber alternativ auch lösbar mit dem Prozessor 503 verbunden sein, beispielsweise als Speicherkarte, Diskette, oder optisches Datenträgermedium (z.B. eine CD oder DVD). In dem Programmspeicher 502, oder in einem separaten Speicher, können auch weitere Informationen abgespeichert sein.

Der Arbeitsspeicher 501 wird beispielsweise zur Speicherung temporärer Ergebnisse während der Abarbeitung der Programmanweisungen genutzt, es handelt sich hierbei beispielsweise um flüchtigen Speicher, wie beispielsweise einen Random-Access-Memory (RAM)-Speicher.

Der Prozessor 503 ist ferner operativ mit einer Kommunikationsschnittstelle 504 verbunden, mit der beispielsweise ein Informationsaustausch mit anderen Vorrichtungen möglich ist (siehe z.B. die gestrichelten Pfeile in Fig. 1).

Die Vorrichtung 50 kann weitere Komponenten enthalten. Falls die Vorrichtung 50 das Dosiergerät 1 nach Fig. 1 repräsentiert, ist insbesondere eine Sensoreinheit 505 vorgesehen, die beispielsweise zur Erfassung einer Geruchsbelastung im Innenraum eines Reinigungsgerätes der ersten Informationen eingerichtet ist und mit dem Prozessor 503 operativ verbunden ist. Ferner ist insbesondere ein Aktuator 507 vorgesehen, der beispielsweise zum Auslösen einer Beduftung und/oder Desodorierung von Beduftungs- und/oder Desodorierungsmitteln eingerichtet ist und mit dem Prozessor 503 operativ verbunden ist.

Optional kann die Vorrichtung 50 eine Benutzerschnittstelle 506 aufweisen, mittels der beispielsweise eine Wiedergabe von Informationen (z.B. eine optische Wiedergabe) möglich ist. Beispielsweise ist die Benutzerschnittstelle eine Displayvorrichtung (z.B. ein Liquid Crystal Display (LCD), oder ein Light Emitting Diode (LED)-Display oder dergleichen).

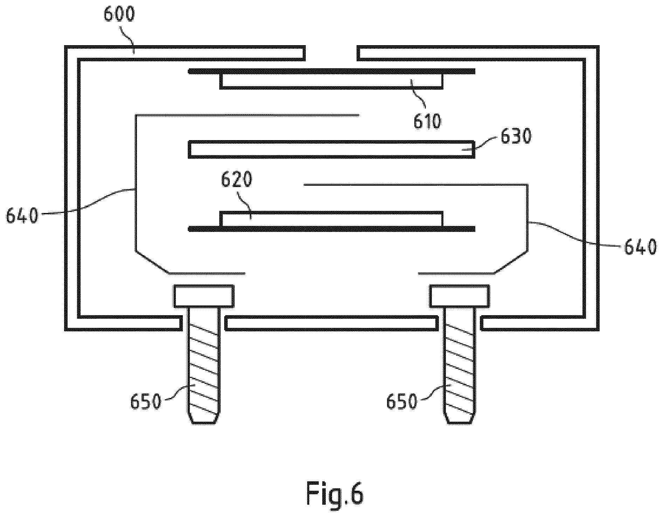

Fig. 6 zeigt eine schematische (Schnitt-) Darstellung eines Aufbaus von einem Geruchssensor, vorliegend ein elektrochemischer Sensor mit der Bezeichnung "SpecSensors 3SP Ethanol 1000 Package 110-202". Mittels dieses dargestellten Geruchssensors kann spezifisch Ethanol erfasst (z.B. detektiert) werden.

Der Geruchssensor 600 umfasst eine Messelektrode 610 und eine Gegenelektrode 620, die voneinander durch einen Separator 630 getrennt sind. Jeweils zumindest teilweise die Elektroden 610, 620 umhüllend ist ein Stromabnehmer 640 angeordnet. Die jeweiligen Stromabnehmer 640 sind zu den jeweiligen Elektroden (Messelektrode 610 und Gegenelektrode 620) beabstandet. Der Geruchssensor 600 umfasst weiterhin zwei Sensorpins 650.

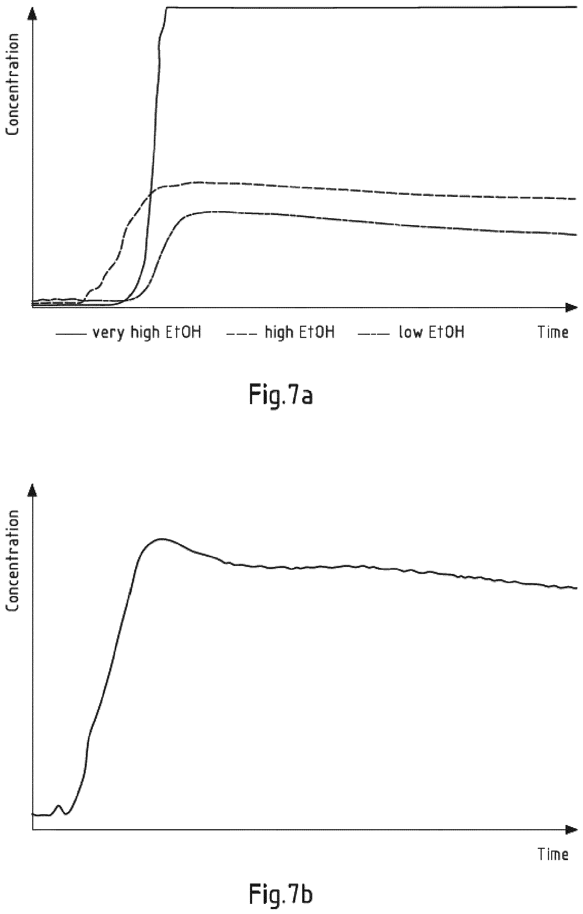

Die Fig. 7a-f zeigen beispielhafte Diagramme von von einem Geruchssensor erfassten (ersten) Informationen. Vorliegend sind den Fig. 7a-f beispielhafte elektrische Signale zu entnehmen, die von einem als Ethanolsensor ausgebildeten Geruchssensor erzeugt wurden.

789 mg, 78,9 mg und 7,9 mg Ethanol werden jeweils in den Innenraum einer Geschirrspülmaschine eingebracht. Vorliegend weist die Geschirrspülmaschine ein Behandlungsvolumen von 183,6 I auf. Das Ethanol wird mittels einer Pipette in den Innenraum eingebracht. Entsprechend der Dampfdrucke ergeben sich maximale Konzentrationen von 4300 ppm (very high EtOH), 430 ppm (high EtOH) bzw. 43m ppm (low EtOH) in der Gasphase. Im Innenraum der Geschirrspülmaschine ist ein Geruchssensor, vorliegend ein elektrochemischer Sensor des Typs "SpecSensors 3SP Ethanol 1000 Package 110-202", angeordnet zur Erfassung des eingebrachten Ethanols.

Fig. 7a zeigt das von einem Geruchssensor, vorliegend ein in dieser Spezifikation beschriebener Ethanolsensor, erfasste elektrische Signal. Vorliegend sind erfasste unterschiedliche Ethanol-Konzentrationen über einen Zeitraum abgebildet. Die Erfassung der Ethanol-Konzentrationen erfolgte in einer geschlossen, nicht gelüfteten Geschirrspülmaschine. Aus den jeweiligen Graphen ist ersichtlich, dass auch sehr kleine Konzentrationen (low EtOH = 43 ppm), wie sie bei biologischen Zersetzungsvorgängen entstehen, mittels des Ethanolsensors erfasst werden können. Überraschenderweise wurde herausgefunden, dass mittels eines Ethanolsensors auch komplexere Alkohol-Moleküle erfassbar sind.

Fig. 7b zeigt ein beispielhaftes Diagramm von von einem Geruchssensor erfassten (ersten) Informationen, die vorliegend eine iso-Propanol Konzentration repräsentieren.

Der in Fig. 7b dargestellte Graph zeigt den Konzentrations-Zeit-Verlauf einer Dosierung von 39 mg 2-Propanol in einer geschlossen, nicht gelüfteten Geschirrspülmaschine entsprechend einer Konzentration von 212 ppm. Die Sensorantwort auf diese erfasste Konzentration, erfasst als elektrisches Signal) ist deutlich zu erkennen.

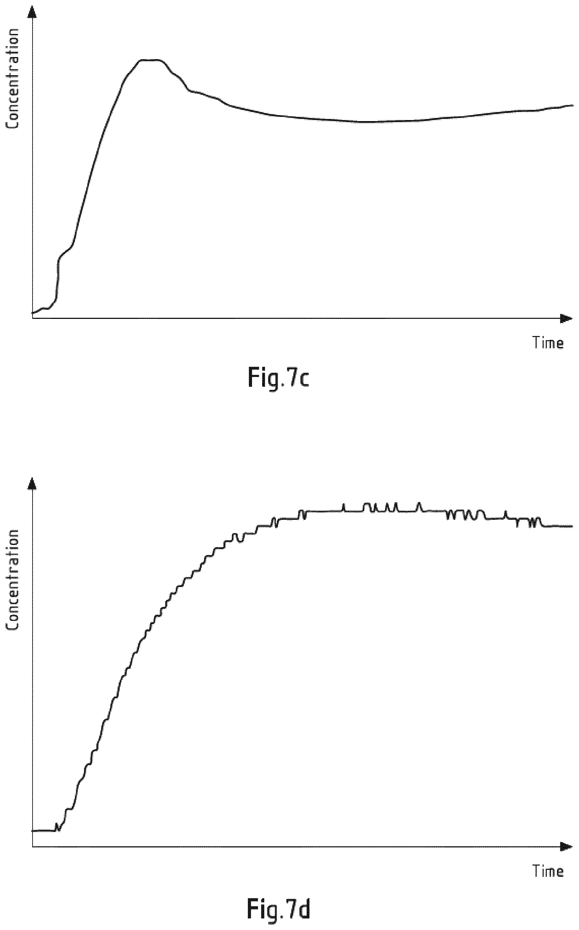

Fig. 7c zeigt ein beispielhaftes Diagramm von von einem Geruchssensor erfassten (ersten) Informationen, die vorliegend eine 1-Butanol Konzentration repräsentieren.

Der in Fig. 7c dargestellte Graph zeigt den Konzentrations-Zeit-Verlauf einer Dosierung von 40,5 mg 1-Butanol in einer geschlossen, nicht gelüfteten Geschirrspülmaschine entsprechend einer Konzentration von 220 ppm. Die Sensorantwort auf diese erfasste Konzentration, erfasst als elektrisches Signal) ist deutlich zu erkennen.

Fig. 7d zeigt ein beispielhaftes Diagramm von von einem Geruchssensor erfassten (ersten) Informationen, die vorliegend eine Ethylacetat Konzentration repräsentieren.

Der in Fig. 7d dargestellte Graph zeigt den Konzentrations-Zeit-Verlauf einer Dosierung von 44,7 mg Ethylacetat in einer geschlossen, nicht gelüfteten Geschirrspülmaschine entsprechend einer Konzentration von 243 ppm. Die Sensorantwort auf diese erfasste Konzentration, erfasst als elektrisches Signal) ist deutlich zu erkennen.

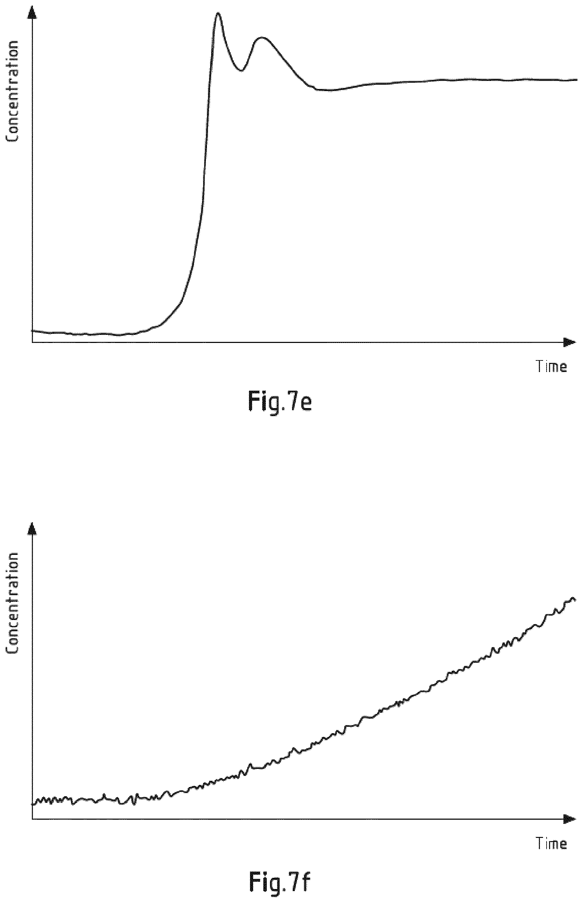

Fig. 7e zeigt ein beispielhaftes Diagramm von von einem Geruchssensor erfassten (ersten) Informationen, die vorliegend eine i-Butyraldehyd Konzentration repräsentieren.

Der in Fig. 7e dargestellte Graph zeigt den Konzentrations-Zeit-Verlauf einer Dosierung von 39,5 mg i-Butyraldehyd in einer geschlossen, nicht gelüfteten Geschirrspülmaschine entsprechend einer Konzentration von 215 ppm. Die Sensorantwort auf diese erfasste Konzentration, erfasst als elektrisches Signal) ist deutlich zu erkennen.

Fig. 7f zeigt ein beispielhaftes Diagramm von von einem Geruchssensor erfassten (ersten) Informationen, die vorliegend eine 2-Propanon (Aceton) Konzentration repräsentieren.

Der in Fig. 7f dargestellte Graph zeigt den Konzentrations-Zeit-Verlauf einer Dosierung von 395 mg 2-Propanon (Aceton) in einer geschlossen, nicht gelüfteten Geschirrspülmaschine entsprechend einer Konzentration von 2151 ppm. Die Sensorantwort auf diese erfasste Konzentration, erfasst als elektrisches Signal) ist zu erkennen, aber vergleichsweise zu den Graphen gemäß der Fig. 7b-e schwach ausgebildet.

Die in dieser Spezifikation beschriebenen Ausführungsbeispiele und die diesbezüglich jeweils angeführten optionalen Merkmale und Eigenschaften sollen auch in allen Kombinationen miteinander offenbart verstanden werden. Insbesondere soll auch die Beschreibung eines von einem Ausführungsbeispiel umfassten Merkmals - sofern nicht explizit gegenteilig erklärt - vorliegend nicht so verstanden werden, dass das Merkmal für die Funktion des Ausführungsbeispiels unerlässlich oder wesentlich ist. Die Abfolge der in dieser Spezifikation geschilderten Verfahrensschritte in den einzelnen Ablaufdiagrammen ist nicht zwingend, alternative Abfolgen der Verfahrensschritte sind denkbar. Die Verfahrensschritte können auf verschiedene Art und Weise implementiert werden, so ist eine Implementierung in Software (durch Programmanweisungen), Hardware oder eine Kombination von beidem zur Implementierung der Verfahrensschritte denkbar.

In den Patentansprüchen verwendete Begriffe wie "umfassen", "aufweisen", "beinhalten", "enthalten" und dergleichen schließen weitere Elemente oder Schritte nicht aus. Unter die Formulierung "zumindest teilweise" fallen sowohl der Fall "teilweise" als auch der Fall "vollständig". Die Formulierung "und/oder" soll dahingehend verstanden werden, dass sowohl die Alternative als auch die Kombination offenbart sein soll, also "A und/oder B" bedeutet "(A) oder (B) oder (A und B)". Die Verwendung des unbestimmten Artikels schließt eine Mehrzahl nicht aus. Eine einzelne Vorrichtung kann die Funktionen mehrerer in den Patentansprüchen genannten Einheiten bzw. Vorrichtungen ausführen. In den Patentansprüchen angegebene Bezugszeichen sind nicht als Beschränkungen der eingesetzten Mittel und Schritte anzusehen.

## Patentansprüche

1. Verfahren durchgeführt von einer oder mehreren Vorrichtungen (1, 2, 3, 50, 51), umfassend:
- Erfassen von ersten Informationen oder Erhalten von erfassten ersten Informationen indikativ für eine Geruchsbelastung im Innenraum eines Reinigungsgerätes (2), wobei die ersten Informationen von mindestens einem Geruchssensor erfasst werden;
- Erzeugen einer Beduftungs- und/oder Desodorierungsinformation zumindest teilweise basierend auf den erfassten oder erhaltenen ersten Informationen oder Ausgeben der erfassten ersten Informationen zum Erzeugen einer derartigen Beduftungs- und/oder Desodorierungsinformation;
- Auslösen einer Beduftung und/oder Desodorierung mittels mindestens eines Aktuators (13, 507) für Beduftungs- und/oder Desodorierungsmittel basierend auf der erzeugten Beduftungs- und/oder Desodorierungsinformation,
**dadurch gekennzeichnet, dass** die erfassten ersten Informationen indikativ für zumindest die Dynamik der Geruchsbelastung im Innenraum des Reinigungsgerätes sind.

2. Verfahren nach Anspruch 1, wobei das Erzeugen der Beduftungs- und/oder Desodorierungsinformation einen Vergleich zwischen den erfassten ersten Informationen mit einem vordefinierten Schwellwert umfasst.

3. Verfahren nach einem der vorhergehenden Ansprüche, wobei die erfassten ersten Informationen und/oder der vordefinierte Schwellwert indikativ für zumindest einen der folgenden Parameter sind:
- Intensität der Geruchsbelastung im Innenraum des Reinigungsgerätes;
- Art der Geruchsbelastung im Innenraum des Reinigungsgerätes;
- oder eine Kombination hiervon.

4. Verfahren nach Anspruch 2 oder Anspruch 3, das Verfahren umfassend:
- Erhalten einer Nutzereingabe betreffend eine Anpassung des vordefinierten Schwellwertes;
- Adaptieren des vordefinierten Schwellwertes basierend auf der erhaltenen Nutzereingabe.

5. Verfahren nach einem der vorhergehenden Ansprüche, das Verfahren umfassend:
- Erfassen von zweiten Informationen oder Erhalten von erfassten zweiten Informationen indikativ für einen Lichteintritt und/oder eine Temperatur im Innenraum des Reinigungsgerätes (2), wobei die zweiten Informationen von mindestens einem Lichtsensor und/oder mindestens einem Temperatursensor erfasst werden.

6. Verfahren nach Anspruch 5, das Verfahren umfassend:
- Adaptieren des vordefinierten Schwellwertes basierend auf den erfassten zweiten Informationen, insbesondere ein Adaptieren des vordefinierten Schwellwertes mittels eines Nullpunkt-Kalibrierens im Fall, dass die Temperatur einen vordefinierten Temperaturschwellwert unterschritten und der Lichteintritt ein Ausräumen des Reinigungsgerätes (2) signalisiert hat.

7. Verfahren nach Anspruch 5 oder Anspruch 6, wobei der mindestens eine Geruchssensor und der mindestens eine Lichtsensor und/oder der mindestens eine Temperatursensor ein Sensorarray (11, 505) ausbilden.

8. Verfahren nach einem der vorhergehenden Ansprüche, wobei das Verfahren zwischen zwei Reinigungszyklen des Reinigungsgerätes (2) durchgeführt und/oder gesteuert wird.

9. Verfahren nach einem der vorhergehenden Ansprüche, wobei das Auslösen einer Beduftung und/oder Desodorierung durchgeführt wird, wenn die erzeugte Beduftungs- und/oder Desodorierungsinformation indikativ für eine von einem Nutzer als unangenehm empfundene Geruchsbelastung im Innenraum des Reinigungsgerätes (2) ist.

10. Verfahren nach einem der vorhergehenden Ansprüche, wobei das Auslösen der Beduftung und/oder Desodorierung unterschiedliche Beduftungs- und/oder Desodorierungsmittel, insbesondere in Abhängigkeit der Art der Geruchsbelastung im Innenraum des Reinigungsgerätes (2), freisetzt.

11. Verfahren nach einem der vorhergehenden Ansprüche, wobei das Auslösen der Beduftung und/oder Desodorierung unterschiedliche Mengen des Beduftungs- und/oder Desodorierungsmittels, insbesondere in Abhängigkeit der Intensität der Geruchsbelastung im Innenraum des Reinigungsgerätes (2), freisetzt.

12. Dosiergerät (1) zur Positionierung im Innenraum eines Reinigungsgerätes (2), umfassend:
- mindestens eine Kartusche zur Bevorratung von mindestens einer Zubereitung eines Beduftungs- und/oder Desodorierungsmittels;
- eine Sensoreinheit (11, 505) umfassend mindestens einen Geruchssensor;
- eine Steuereinheit (12, 50), wobei mittels der Steuereinheit eine Beduftungs- und/oder Desodorierungsinformation basierend auf von der Sensoreinheit erfassten ersten Informationen erzeugbar ist;
- einen Aktuator (13, 507) zum Auslösen einer Beduftung und/oder Desodorierung basierend auf der erzeugten Beduftungs- und/oder Desodorierungsinformation, wobei mindestens eine Zubereitung eines Beduftungs- und/oder Desodorierungsmittels freisetzbar ist
- optional eine Kommunikationsschnittstelle,
**dadurch gekennzeichnet, dass** der Geruchssensor eingerichtet ist erste Informationen zu erfassen oder erfasste erste Informationen indikativ für eine Geruchsbelastung im Innenraum des Reinigungsgerätes zu erhalten, wobei die erfassten ersten Informationen indikativ für zumindest die Dynamik der Geruchsbelastung im Innenraum des Reinigungsgerätes sind.

13. Dosiergerät (1) nach Anspruch 12, die Sensoreinheit (11, 505) umfassend:
- mindestens einen Lichtsensor und/oder mindestens einen Temperatursensor.

14. Vorrichtung, welche dazu eingerichtet ist ein Verfahren nach einem der vorhergehenden Ansprüche durchzuführen und/oder zu steuern.

15. System, umfassend:
- mindestens eine Vorrichtung (1, 3, 50, 51) und ein Reinigungsgerät (2), welche zusammen ein Verfahren nach einem der vorhergehenden Verfahrensansprüche durchführen, wobei optional mindestens eine Vorrichtung (1, 2, 3, 50, 51) eine Kommunikationsschnittstelle aufweist.

## Claims

1. Method carried out by one or more devices (1, 2, 3, 50, 51), comprising:
- capturing first information or receiving captured first information indicative of an odor in the interior of a cleaning appliance (2), the first information being captured by at least one odor sensor;
- generating fragrancing and/or deodorizing information at least partially on the basis of the captured or received first information or outputting the captured first information for generating such fragrancing and/or deodorizing information;
- triggering fragrancing and/or deodorizing by means of at least one actuator (13, 507) for fragrancing and/or deodorizing agents on the basis of the generated fragrancing and/or deodorizing information,
**characterized in that** the captured first information is indicative of at least the dynamics of the odor in the interior of the cleaning appliance.

2. Method according to claim 1, wherein the generation of the fragrancing and/or deodorizing information comprises a comparison between the captured first information and a predefined threshold value.

3. Method according to one of the preceding claims, wherein the captured first information and/or the predefined threshold value are indicative of at least one of the following parameters:
- intensity of the odor in the interior of the cleaning apparatus;
- type of odor in the interior of the cleaning apparatus;
- or a combination thereof.

4. Method according to claim 2 or claim 3, the method comprising:
- receiving user input regarding an adjustment of the predefined threshold value;
- adapting the predefined threshold value on the basis of the received user input.

5. Method according to one of the preceding claims, the method comprising:
- capturing second information or receiving captured second information indicative of an incidence of light and/or a temperature in the interior of the cleaning appliance (2), wherein the second information is captured by at least one light sensor and/or at least one temperature sensor.

6. Method according to claim 5, the method comprising:
- adapting the predefined threshold value on the basis of the captured second information, in particular adapting the predefined threshold value by means of a zero point calibration in the event that the temperature has fallen below a predefined temperature threshold value and the incidence of light has signaled that the cleaning apparatus (2) has been cleared out.

7. Method according to claim 5 or claim 6, wherein the at least one odor sensor and the at least one light sensor and/or the at least one temperature sensor form a sensor array (11, 505).

8. Method according to one of the preceding claims, wherein the method is carried out and/or controlled between two cleaning cycles of the cleaning apparatus (2).

9. Method according to one of the preceding claims, wherein fragrancing and/or deodorizing is triggered if the generated fragrancing and/or deodorizing information is indicative of an odor in the interior of the cleaning apparatus (2) that a user finds unpleasant.

10. Method according to one of the preceding claims, wherein triggering the fragrancing and/or deodorizing releases different fragrancing and/or deodorizing agents, in particular depending on the type of odor in the interior of the cleaning apparatus (2).

11. Method according to one of the preceding claims, wherein triggering the fragrancing and/or deodorizing releases different amounts of the fragrancing and/or deodorizing agent, in particular depending on the intensity of the odor in the interior of the cleaning apparatus (2).

12. Dosing device (1) to be positioned in the interior of a cleaning apparatus (2), comprising:
- at least one cartridge for storing at least one preparation of a fragrancing and/or deodorizing agent;
- a sensor unit (11, 505) comprising at least one odor sensor;
- a control unit (12, 50), it being possible for the control unit to generate fragrancing and/or deodorizing information on the basis of first information captured by the sensor unit;
- an actuator (13, 507) for triggering fragrancing and/or deodorizing on the basis of the generated fragrancing and/or deodorizing information, it being possible for at least one preparation of a fragrancing and/or deodorizing agent to be released;
- optionally a communication interface;
**characterized in that** the odor sensor is designed to capture first information or to receive captured first information indicative of an odor in the interior of the cleaning apparatus, the captured first information being indicative of at least the dynamics of the odor in the interior of the cleaning apparatus.

13. Dosing device (1) according to claim 12, the sensor unit (11, 505) comprising:
- at least one light sensor and/or at least one temperature sensor.

14. Device which is designed to carry out and/or control a method according to one of the preceding claims.

15. System comprising:
- at least one device (1, 3, 50, 51) and a cleaning apparatus (2), which together carry out a method according to one of the preceding method claims, wherein optionally at least one device (1, 2, 3, 50, 51) has a communication interface.

## Revendications

1. Procédé exécuté par un ou plusieurs dispositifs (1, 2, 3, 50, 51), comprenant :
l'acquisition de premières informations ou la réception de premières informations acquises indiquant une nuisance olfactive dans l'espace intérieur d'un appareil de nettoyage (2), les premières informations étant acquises par au moins un capteur d'odeurs ;
la génération d'informations de parfumage et/ou de désodorisation au moins partiellement sur la base des premières informations acquises ou reçues ou la sortie des premières informations acquises pour générer de telles informations de parfumage et/ou de désodorisation ;
le déclenchement d'un parfumage et/ou d'une désodorisation au moyen d'au moins un actionneur (13, 507) d'agents de parfumage et/ou de désodorisation sur la base des informations de parfumage et/ou de désodorisation générées,
**caractérisé en ce que** les premières informations acquises indiquent au moins la dynamique de la nuisance olfactive dans l'espace intérieur de l'appareil de nettoyage.

2. Procédé selon la revendication 1, dans lequel la génération des informations de parfumage et/ou de désodorisation comprend une comparaison entre les premières informations acquises et une valeur seuil prédéfinie.

3. Procédé selon l'une des revendications précédentes, dans lequel les premières informations acquises et/ou la valeur seuil prédéfinie indiquent au moins l'un des paramètres suivants :
intensité de la nuisance olfactive dans l'espace intérieur de l'appareil de nettoyage ; type de la nuisance olfactive dans l'espace intérieur de l'appareil de nettoyage ; ou leur combinaison.

4. Procédé selon la revendication 2 ou la revendication 3, le procédé comprenant :
la réception d'une entrée utilisateur concernant un ajustement de la valeur seuil prédéfinie ;
l'adaptation de la valeur seuil prédéfinie sur la base de l'entrée utilisateur reçue.

5. Procédé selon l'une des revendications précédentes, le procédé comprenant :
l'acquisition de secondes informations ou la réception de secondes informations acquises indiquant une entrée de lumière et/ou une température dans l'espace intérieur de l'appareil de nettoyage (2), les secondes informations étant acquises par au moins un capteur de lumière et/ou au moins un capteur de température.

6. Procédé selon la revendication 5, le procédé comprenant :
l'adaptation de la valeur seuil prédéfinie sur la base des secondes informations acquises, en particulier l'adaptation de la valeur seuil prédéfinie au moyen d'un étalonnage de point zéro dans le cas où la température est tombée en dessous d'une valeur seuil de température prédéfinie et que l'entrée de lumière a signalé que l'appareil de nettoyage (2) a été vidé.

7. Procédé selon la revendication 5 ou la revendication 6, dans lequel l'au moins un capteur d'odeurs et l'au moins un capteur de lumière et/ou l'au moins un capteur de température forment un réseau de capteurs (11, 505).

8. Procédé selon l'une des revendications précédentes, dans lequel le procédé est exécuté et/ou commandé entre deux cycles de nettoyage de l'appareil de nettoyage (2).

9. Procédé selon l'une des revendications précédentes, dans lequel le déclenchement d'un parfumage et/ou d'une désodorisation est exécuté lorsque les informations de parfumage et/ou de désodorisation générées indiquent une nuisance olfactive dans l'espace intérieur de l'appareil de nettoyage (2) qu'un utilisateur trouve désagréable.

10. Procédé selon l'une des revendications précédentes, dans lequel le déclenchement du parfumage et/ou de la désodorisation libère différents agents de parfumage et/ou de désodorisation, en particulier en fonction du type de la nuisance olfactive dans l'espace intérieur de l'appareil de nettoyage (2).

11. Procédé selon l'une des revendications précédentes, dans lequel le déclenchement du parfumage et/ou de la désodorisation libère différentes quantités de l'agent de parfumage et/ou de désodorisation, en particulier en fonction de l'intensité de la nuisance olfactive dans l'espace intérieur de l'appareil de nettoyage (2).

12. Appareil de dosage (1) à positionner dans l'espace intérieur d'un appareil de nettoyage (2), comprenant :
au moins une cartouche permettant de stocker au moins une préparation d'un agent de parfumage et/ou de désodorisation ;
une unité de capteur (11, 505) comprenant au moins un capteur d'odeurs ;
une unité de commande (12, 50), l'unité de commande pouvant générer des informations de parfumage et/ou de désodorisation sur la base de premières informations acquises par l'unité de capteur ;
un actionneur (13, 507) permettant de déclencher un parfumage et/ou une désodorisation sur la base des informations de parfumage et/ou de désodorisation générées, au moins une préparation d'un agent de parfumage et/ou de désodorisation pouvant être libérée
éventuellement une interface de communication,
**caractérisé en ce que** le capteur d'odeurs est configuré pour acquérir des premières informations ou pour recevoir des premières informations acquises indiquant une nuisance olfactive dans l'espace intérieur de l'appareil de nettoyage, les premières informations acquises indiquant au moins la dynamique de la nuisance olfactive dans l'espace intérieur de l'appareil de nettoyage.

13. Appareil de dosage (1) selon la revendication 12, l'unité de capteur (11, 505) comprenant :
au moins un capteur de lumière et/ou au moins un capteur de température.

14. Dispositif, lequel est configuré pour exécuter et/ou commander un procédé selon l'une des revendications précédentes.

15. Système, comprenant :
au moins un dispositif (1, 3, 50, 51) et un appareil de nettoyage (2), qui exécutent ensemble un procédé selon l'une des revendications de procédé précédentes,
éventuellement au moins un dispositif (1, 2, 3, 50, 51) présentant une interface de communication.
